# NEUE EUROPÄISCHE PATENTSCHRIFT

(11) **EP 2 002 864 B2**
(45) Veröffentlichungstag und Bekanntmachung des Hinweises auf die Entscheidung über den Einspruch: **14.01.2015**
(45) Hinweis auf die Patenterteilung: 25.07.2012
(21) Anmeldenummer: 08016946.9
(22) Anmeldetag: 27.08.2004
(51) Int. Cl.: A61K 8/06, A61K 8/31, A61K 8/34, A61K 8/37, A61K 8/11, A61K 8/92, A61Q 19/00

(54) **Kosmetische und/oder dermatologische Kapsel**
Cosmetic and/or dermatological capsule
Capsule à usage cosmétique et/ou dermatologique

(30) Priorität: 27.08.2003 DE 10339747; 30.08.2003 DE 10340106; 10.12.2003 DE 10357639
(43) Veröffentlichungstag der Anmeldung: 17.12.2008
(62) Teilanmeldung aus: 04764537.9
(73) Patentinhaber: Beiersdorf AG, 20245 Hamburg (DE)
(72) Erfinder: Kallmayer, Volker, 22393 Hamburg (DE); Raschke, Thomas, 25421 Pinneberg (DE); Nielsen, Jens, 24558 Henstedt-Ulzburg (DE); Bleckmann, Andreas, 22962 Ahrensburg (DE); Riedel, Heidi, 22083 Hamburg (DE)
(74) Vertreter: Hartmann, Jost

(56) Entgegenhaltungen:
- EP-A- 0 234 078
- EP-A- 1 129 771
- EP-A- 1 201 219
- EP-A- 1 473 016
- WO-A-00/10522
- WO-A-01/03538
- DE-A- 4 223 004
- DE-A- 10 044 062
- US-A1- 2002 022 038
- DATABASE WPI Section Ch, Week 199837 Thomson Scientific, London, GB; Class A26, AN 1998-433672 XP002313336 & JP 10 182337 A (NOEVIR KK) 7. Juli 1998 (1998-07-07)
- DATABASE WPI Section Ch, Week 200355 Thomson Scientific, London, GB; Class A14, AN 2003-580900 XP002313041 & JP 2003 073230 A (SHISEIDO CO LTD) 12. März 2003 (2003-03-12)
- DATABASE WPI Section Ch, Week 200227 Thomson Scientific, London, GB; Class A96, AN 2002-211272 XP002313250 & JP 2001 348310 A (KANEBO LTD) 18. Dezember 2001 (2001-12-18)

## Beschreibung

Die Erfindung umfasst kosmetische und dermatologische Zubereitungen in Form von Kapseln umfassend eine Kapselhülle, die fest, halbfest und/oder formstabil ist und im Wesentlichen aus Wachsen, Emulgatoren, natürlichen und/oder synthetischen Polymeren und/oder Mischungen daraus besteht, sowie das Verfahren zu ihrer Herstellung. Des Weiteren umfasst die Erfindung eine Kapsel mit einer Füllung bestehend aus einem oder mehreren festen, halbfesten, pastösen und/oder flüssigen Inhaltsstoffen. Die Hülle enthält zusätzlich Wasser und kann optional Polyole enthalten.

Die Eigenschaft der Hülle ist charakterisiert dadurch, dass sie
- beim Verreiben und/oder Verteilen der Zubereitung auf der Haut und/oder dem Haar schmilzt und/oder aufgrund von Scherkräften völlig oder teilweise flüssig wird
- und/oder sie sich in der Füllung und/oder den Hautsebumlipiden bzw. durch Vermischung von innerer Phase und Hüllmaterial auflöst
und so für den Anwender nicht mehr als gesondert neben der Füllung vorliegender Bestandteil der Zubereitung wahrnehmbar ist.

Aus dem Stand der Technik sind pharmazeutische Kapseln, beispielsweise Weich- oder Hartgelantinekapseln bekannt, die aus einer Hülle aus Gelantine und Glycerol sowie gelegentlich Farbstoffen, und einer flüssigen, pastösen oder partikulären Füllung bestehen. Das Verfahren zur Herstellung dieser seit vielen Jahren bekannten Kapseln erfolgt z.B. nach dem sog. Rotary-Die-System (Seifen-Öle-Fette-Wachse, 113. Jg; Nr 3/1987, Seite 67 ff.). Diese Gelantinekapseln lösen sich beim Verschlucken im Magen-Darmtrakt auf und geben ihre Inhaltstoffe frei. Da es ohne weiteres möglich ist, Kapseln in unterschiedlichen Größen und Formen zu erzeugen, ist die Anwendung nicht nur auf auf perorale Applikationsformen beschränkt (Rudolf Voigt, "Pharmazeutische Technologie", Deutsche Apotheker Verlag, Stuttgart, 9. Auflage (2000), S.543ff). Eine orale Resorption kann durch Lutschkapseln erzielt werden. Sie sind innen hohl und besitzen eine dreifach stärkere Wanddicke als andere Kapseln. Der Wirkstoff ist hierbei in der Gelantinehülle eingearbeitet. Ein weiteres Beispiel sind Nitroglycerin-Kaukapseln (Zerbeißkapseln), die ebenfalls eine rasche Resorption des Wirkstoffs über die Mundschleimhaut ermöglichen. Des Weiteren können einzeldosierte Arzneistoffe nach Aufstechen oder Aufschneiden der tubenförmigen Salbenkapseln durch Ausquetschen des Inhalts zur Applikation gebracht werden (perkutane Applikation von Nitroglycerin-Herzsalbe).

Aus dem Lebensmittelsbereich sind beispielsweise Pralinees bekannt, die in einer formstabilen Schokoladenhülle flüssige oder pastöse Inhaltsstoffe enthalten. Die Hülle schmilzt beim Lutschen oder Zerbeißen im Mund oder nach dem Verschlucken.

Im Kosmetikbereich sind die sogenannten Badeperlen bekannt, deren Hülle, z.B aus Gelatine sich im heißen oder warmen Badewasser rückstandsfrei auflöst und ihren Inhalt, beispielsweise Tensidzubereitungen, Emulsionen, Lipide, Farbstoffe und/oder Parfume, ins Badewasser freigibt. Da die Hülle aus Gelatine besteht, darf das Produkt kein Wasser enthalten, andernfalls würde die Hülle während der Lagerung aufweichen.

Eine zweite Gruppe kosmetischer Kapseln umfasst alle Produkte, für welche die Kapselhülle nur ein Behältnis zur einmaligen Dosierung und Anwendung darstellt und deren Hülle nach der Anwendung übrig bleibt. Nachteilig ist hieran, dass die übrig bleibende Hülle störend wirkt und zudem entsorgt werden muss.

Zahlreiche kosmetische und/oder dermatologische Wirkstoffe sind gegenüber bestimmten Einflüssen wie Feuchtigkeit, niedrigen oder hohen pH-Werten sowie Sauerstoff oder Licht nicht stabil. Es hat daher nicht an Versuchen gefehlt, derartige Wirkstoffe den genannten unerwünschten Umwelteinflüssen so zu entziehen, dass dennoch bei der Anwendung eine Freisetzung der Wirkstoffe stattfindet. Ein Weg zur Erreichung dieses Ziels ist die Mikro- oder Nanoverkapselung von Wirkstoffen. Das Verkapselungsmaterial als Trägersystem für die Wirkstoffe erlaubt dabei, sie vor Umwelteinflüssen geschützt in geeignete Zubereitungen einzuarbeiten, ohne dass der Verwender die Kapseln bei der Produktapplikation wahrnehmen könnte.
Ziel einer solchen Verkapselung ist es beispielsweise, wirkstoffhaltige Wachspartikel im Mikrometerbereich (1 - 250 µm) herzustellen, die in gängigen pastösen oder flüssigen kosmetischen Zubereitungen einzuarbeiten sind. Es ist bisher nicht bekannt, diese Mikrokapseln als eigenständige kosmetische Zubereitung herzustellen, zu lagern und topisch anzuwenden. In der DE 10210449 werden wachsbeschichtete Kapseln, die mit Wirkstoff beladen sind, offenbart. Diese Kapseln werden mittels eines so genannten Wirbelschichtverfahrens hergestellt, so dass maximal Kapseln mit einer Größe von 200 µm hergestellt werden können.

Zur Verkapselung kosmetischer Wirkstoffe gibt es mehrere Ansätze. Bekannt ist beispielsweise die liposomale Verkapselung von Arzneistoffen, die zu einer retardierten Wirkstofffreisetzung führen soll. Im Wesentlichen handelt es sich dabei um von Phospholipiden oder anderen Amphiphilen umschlossene wirkstoffhaltige sphärische Vesikel, die sogenannten Liposome. Die Langzeitstabilität derartiger Gebilde ist jedoch gering. Nanopartikel sind feste Partikel mit Partikelgrößen von 20 bis 500 nm. Gelegentlich werden auch größere Teilchen mit Durchmessern bis zu 1000 nm zu den Nanopartikeln gerechnet. Derartige Partikel bestehen in der Regel aus Polymeren und weisen Kavitäten auf oder bilden eine Hülle, so dass sich in ihrem Inneren Gastmoleküle aufhalten können, die umschlossen oder adsorbiert werden. Diese Gastmoleküle werden dann bei der Anwendung des Nanopartikel enthaltenden Produktes langsam freigesetzt. Ähnlich verhalten sich feste Lipidnanopartikel, die in einer Matrix aus festen Lipiden verteilte Wirkstoffe enthalten. Die Größe der Partikel ist mit denen der Nanopartikel vergleichbar.

Zur Verkapselung von pharmazeutischen oder kosmetischen Wirkstoffen zur Kontrolle der Wirkstofffreisetzung oder der stabilen Einarbeitung in Zubereitungen sind zahlreiche Methoden bekannt.
Die europäische Patentanmeldung EP 1064911 bzw. EP 1064912 offenbart Wirkstoff enthaltende Mikrokapseln mit einem Durchmesser von 0,1 bis 5 mm, die man erhält, in dem man aus Gelbildnern, Chitosan und Wirkstoff eine Matrix herstellt, und diese in wässrige Lösungen anionischer Polymere eintropft. Dabei bildet sich aus Chitosan und anionischem Polymer eine Membran, die die Wirkstofflösung umhüllt. Diese Mikropartikel werden dann wiederum als Bestandteil gängiger kosmetischer Zubereitungen verwendet. Allgemeines zu Verkapselungstechniken mit Chitosan findet sich in Journal of Microencapsulation 14, Seiten 689-711 (1997).

In Lipidpartikeln verkapselte, zumeist lipophile Wirkstoffe sind an sich dem Fachmann bekannt. So beschreiben die EP 167825, DE 100 59 668, DE 199 45 203, EP 0934743, WO 94/20072, WO 00/10522 sowie die WO 00/67728 wirkstoffbeladene Lipidpartikel. Doch konnten diese Schriften nicht das Problem der Bereitstellung von kapselförmigen Zubereitungen mit umschlossenen flüssigen, pastösen oder festen kosmetischen Inhaltsstoffen lösen, die als eigenständige kosmetische Zubereitung herzustellen, zu lagern und topisch anzuwenden sind.

In der WO 00/10522 A werden wässrige Zubereitungen offenbart, die mikroskopisch kleine Wachspartikel mit einer Größe < 500 nm umfassen. Die Partikel können zwar aus unterschiedlichen Rohstoffen, wie Wachse, Emulgatoren etc. aufgebaut sein, jedoch liegen sie immer als einheitliche zusammenhängende Matrix vor.

WO 01/03538 A offenbart keine einzeln handhabbaren Kapseln mit einem Durchmesser von mehr als 5 mm. Ebenso umfassen die Kapseln keine Füllung. Die beschriebenen Kapseln können nicht direkt auf die Haut aufgetragen werden. Ihre Hülle ist für eine manuelle Entnahme zu dünn, weshalb sie durch eine Stoffschicht "ausgedrückt" und appliziert werden sollen.

Der Durchmesser der in US 2002/022038 A1 offenbarten Kapseln beträgt max. 1000 µm, bevorzugt 15 - 25 µm.

EP 0 234 078 A1 beschreibt Mikrokapseln mit einer Größe bis 5 mm. Die Kapselhülle ist wasserfrei.

EP 1129771 A1 beschreibt Mikrokapseln mit einer Kapselgröße bis 5 mm und einer Chitosanhülle.

DE 4223004 A2 offenbart einzeldosierte halbfeste topische Arzneiformen. Zur Verbesserung der Handhabbarkeit können diese in Weichgelatinekapseln umhüllt werden.

Aus DE 19852262, DE 9321186 und CH 692968 sind Verfahren zur Herstellung von Süssmassen, insbesondere Schokoladenartikeln, nach dem sog. one-shot, frozen-cone bzw. cold-stamp Verfahren bekannt.

Die hierin beschriebenen Verfahren befassen sich ausschließlich mit der Herstellung von Schokoladenartikeln, Pralinés.

Aufgabe der vorliegenden Erfindung ist es, eine kosmetische Zubereitung bereit zu stellen, die als feste, halbfeste bzw. formstabile Kapsel vorliegt, damit einzeln portionierbar ist und als Ganzes auf der Haut verteilt werden kann. Insbesondere ist es die Aufgabe der vorliegenden Erfindung eine kosmetische Zubereitung bereit zu stellen, die eine neue kosmetische Produktform darstellt und dem Anwender ein neues Applikationserlebnis bietet und das Anwendungsspektrum von Haut- und/oder Haarpflegeprodukten erweitert.

Gelöst wird dieses Bündel an Aufgaben durch eine kosmetische und/oder dermatologische Zubereitung in Kapselform entsprechend den Hauptansprüchen. In den Unteransprüchen sind bevorzugte Ausführungsformen der Zubereitung offenbart. Die Erfindung umfasst darüber hinaus auch das Verfahren zur Applikation der Kapseln auf der Haut und/oder dem Haar, das Verfahren zu ihrer Herstellung und die Kombination mit einer Blisterverpackung und Schachteln zu einem kosmetischen oder dermatologischen Produkt.

Es war überraschend und für den Fachmann außerordentlich erstaunlich, dass die gestellten Aufgaben gelöst werden können durch eine kosmetische und/oder dermatologische Kapsel für kosmetische und/oder dermatologische Inhaltsstoffe umfassend eine Kapselhülle, die fest, halbfest und/oder formstabil ist und im wesentlichen aus Wachsen, Emulgatoren, natürlichen und/oder synthetischen Polymeren und/oder Mischungen daraus besteht, wobei die Polymeren aus der Gruppe Celluloseether, Polyvinylpyrrolidon, Polyacrylate, Polymethacrylate, Polyethylene, Nylon und Eudragit ausgewählt sind.

Die Kapsel umfasst eine Füllung bestehend aus einem oder mehreren festen, halbfesten, pastösen und/oder flüssigen Inhaltsstoffen. Weiter bevorzugt ist, dass die Kapselhülle bis zu einer Temperatur von mindestens 35°C fest, halbfest und/oder formstabil Hülle ist.

Die Kapselhülle enthält zusätzlich Wasser und optional Polyole.

Der Vorteil und gleichzeitig die erfindugsgemäße Eigenschaft der Kapsel ist, dass sie
- beim Verreiben und/oder Verteilen der Kapsel auf der Haut und/oder dem Haar schmilzt und/oder
- aufgrund von Scherkräften völlig oder teilweise flüssig wird und/oder
- sich in der Füllung und/oder den Hautsebumlipiden bzw. durch Vermischung von Füllung und Hüllmaterial auflöst
und so, insbesondere für den Anwender, nicht mehr, insbesondere als gesondert neben der Füllung vorliegender Bestandteil, wahrnehmbar ist.
D.h. die Kapsel zieht vorteilhafterweise bei der Applikation auf der Haut oder dem Haar vollständig ein ohne Rückstände zu hinterlassen.
Insbesondere gegenüber den bekannten kosmetischen Kapseln, deren Kapselhülle nach der Anwendung übrig bleibt, kann erfindungsgemäß die Kapselhülle vollständig auf der Haut verbleiben, so dass es z.B. möglich ist, Wirkstoffe in die Hülle einzuarbeiten. Damit kann die Kapselhülle einen aktiven, pflegenden Beitrag in der kosmetischen und dermatologischen Zubereitung leisten und dient nicht nur der Verpackung.

Viele Begriffe wie "Kugeln", "Kapseln" oder "kapselförmige Zubereitung" können grundsätzlich zur Beschreibung der erfindungsgemäßen Kapseln herangezogen werden, jedoch werden diesen Begriffen unter Umständen verschiedene Bedeutungen zugeordnet. Insbesondere die Bedeutung des Begriffes "Kapsel" ist hierin nicht auf die genau definierten Formen, Herstellungsverfahren, Inhaltsstoffe und Anwendungsmöglichkeiten der ebenfalls "Kapseln" genannten pharmazeutischen Präparate beschränkt, schließt diese aber mit ein. Generell ist erfindungsgemäß eine Kapsel ein beispielsweise ungefähr runder oder ellipsoider, von seiner Umgebung klar unterscheidbarer Gegenstand, der bei leichtem Druck und beispielsweise durch Anfassen bei der Entnahme aus einer Verpackung, seine Form nicht ändert. Erfindungsgemäß sind aber auch anderen Formen der Kapseln bzw. der Zubereitungen denkbar, sofern die beanspruchten Merkmale der Kapsel, der Kapselhülle und der Füllung eingehalten werden.

Die erfindungsgemäße Kapsel umfasst vorportionierte kosmetische Produkte, die aus einer festen oder halbfesten Hülle und vorteilhaft einer Füllung bestehen. Der Begriff fest bzw. halbfest definiert erfindungsgemäß den Zustand der Hülle, entsprechend der pharmazeutischen Technologie. Die feste bzw. halbfeste Hülle ist idealerweise auch als formstabil zu bezeichnen. Jedoch ist auch eine Kapsel mit einer, umgangssprachlich weichen, wabbeligen oder gallertartigen Hülle erfindungsgemäß. Die Hülle bricht, schmilzt und/oder löst sich beim Verreiben auf und löst sich ggf. beim Verteilen mit den Inhaltsstoffen der Füllung auf der Haut oder dem Haar auf. Damit ist gewährleistet, dass eine Zubereitung, beispielsweise als Kosmetikum ohne störende Rückstände auf der Haut oder dem Haar appliziert werden kann.

Es verbleiben gegenüber dem Stand der Technik bekannten kapselförmigen Präparaten keinerlei Hüllmaterialbestandteile ungenutzt auf der Haut zurück, die unansehnlich sind, unnötige Kosten und Umweltbelastungen verursachen und darüber hinaus nach der Produktapplikation vom Verbraucher entfernt werden müssen.

Die erfindungsgemäßen Kapseln weisen eine Größe, d.h. durchschnittlichen Durchmesser bis 40 mm auf. Damit sind die Kapseln einzeln handhabbar und anwendbar.

Die erfindungsgemäßen kapselförmigen Zubereitungen sind als Dragees, Kapseln, Kugeln oder Hohlkugeln bei Lagerung und Entnahme formstabil und werden erst beim Verteilen flüssig bzw. lösen sich auf. Dies wird durch die spezielle Kombination des Hüll- bzw. in Kombination mit dem Kapselmaterial erreicht.

Die Kapselhülle muss unter Lagerungsbedingungen, denen kosmetische Produkte üblicherweise ausgesetzt sind, fest, halbfest und/oder formstabil sein, das heißt vorzugsweise die Form der erfindungsgemäßen Kapseln darf sich während der Lagerung nicht durch beispielsweise den Einfluss der Gravitation oder der Temperatur bis zu 35 °C verändern. Idealerweise kleben die Hüllen während der Lagerung auch dann nicht zusammen, wenn sich zwei Kapseln über längere Zeit berühren. Sollte dieses Erfordernis technologisch unpraktisch sein, lässt sich das Problem durch eine individuelle Umverpackung jeder einzelnen Kapsel, ähnlich einer Einzelverpackung (Bonbon-Papier) lösen.

Darüber hinaus muss das Hüllmaterial die Füllung während der Lagerung vor Austrocknung schützen. Dies trifft besonders zu, wenn die Füllung flüchtige Stoffe wie Wasser, kurzkettige Alkohole (z.B. Ethanol, Isopropanol), Parfum- und Riechstoffe oder niedrig siedende Öle (z. B. Cyclomethicon) enthält.

Das Hüllmaterial verbleibt nach der Anwendung vollständig auf der Haut. Erfindungsgemäß ist es von großer Bedeutung, dass alle Rohstoffe, aus denen die Hülle aufgebaut ist, sehr gut hautverträglich sind. Idealerweise leisten sie einen Beitrag zur Hautpflege, beispielsweise dadurch, dass sie die natürliche Hautbarriere stärken und die Haut so vor Austrocknung schützen.

Die Zusammensetzung der Hülle beeinflusst darüber hinaus maßgeblich das Hautgefühl des Verbrauchers bei Anwendung der erfindungsgemäßen Zubereitungen. Deshalb ist es vorteilhaft, die Hülle aus solchen Stoffen aufzubauen, die bei der Anwendung ein angenehmes Hautgefühl bewirken, sowie die sensorischen Eigenschaften der Hülle und der Füllung eng aufeinander abzustimmen.
Die hier beschriebenen technischen und sensorischen Anforderungen an die Hülle gehen deutlich über das übliche Anforderungsspektrum für bekannte kosmetische Zubereitungen hinaus. Deshalb ist es überraschend, dass sich erfindungsgemäße Hüllen mit Rohstoffen, wie sie dem Fachmanne bereits bekannt sind und in der Kosemtik bereits eingesetzt werden, herstellen lassen.

Es sind folgende Prinzipien bei der Kapsel und deren Hülle zu beachten. Einerseits ist es möglich, bei Raumtemperatur flüssige Lipide oder bei Raumtemperatur flüssige Mischungen von Lipiden unterschiedlicher Schmelzbereiche durch Einbau von Wassertröpfchen (Herstellung einer W/O-Emulsion) derart zu verfestigen, dass die resultierende W/O-Emulsion auch über Raumtemperatur hinaus fest genug ist, um daraus eine erfindungsgemäße Hülle herzustellen. Andererseits können Lipide, deren Schmelzpunkt nahe an der Hauttemperatur von 32°C, also zwischen 30°C und 40°C liegt, zur Herstellung einer solchen erfindungsgemäßen Hülle verwendet werden. Drittens kann durch Verwendung geeigneter Wachse, polymerer Verdicker und/oder Gelbildner aus wässrigen und/oder Lipidsystemen eine thixotrope Zubereitung mit hoher Fließgrenze hergestellt werden, die den Anforderungen an die beschriebene Hülle genügt. Dem Fachmanne ist offensichtlich, dass die genannten Ansätze, Verwendung einer W/O- oder O/W-Emulsion, Verwendung von Lipiden mit vorteilhaften Schmelzpunkten, Verwendung thixotroper Systeme mit geeigneter Fließgrenze, beliebig miteinander kombiniert werden können, um die Eigenschaften der Hülle weiter zu optimieren.

Anhand dieser erfindungsgemäßen Vorgaben kann der Fachmann ohne selbst erfinderisch tätig zu werden eine erfindungsgemäße Kapsel herstellen. Die Kapselhülle ist somit durch die Eigenschaft charakterisiert, dass sie
- beim Verreiben und/oder Verteilen der Zubereitung auf der Haut und/oder dem Haar schmilzt und/oder
- aufgrund von Scherkräften völlig oder teilweise flüssig wird und/oder
- sich in der Füllung und/oder den Hautsebumlipiden bzw. durch Vermischung von Füllung und Hüllmaterial auflöst
und so insbesondere für den Anwender nicht mehr als gesondert neben der Füllung vorliegender Bestandteil der Zubereitung wahrnehmbar ist.

Mehrere erfindungsgemäße Kugeln, Hohlkugeln, Dragees oder Kapseln können gemeinsam in einer Packung aus Papier, Metall oder Plastik etc. oder einzeln oder zu mehreren durch weitere dünne Verpackungen ähnlich Bonbon-Papier oder in einer Blisterverpackung voneinander getrennt gelagert werden.

Vorteilhaft ist insbesondere die Kombination der kapselförmigen Zubereitungen mit einer Blisterverpackung, die während der Lagerung die einzelnen Dragees oder Kapseln voneinander trennt und so ein Zusammenfügen einzelner Kapseln durch beispielweise unsachgemäße Behandlung verhindert. Dies kann auch durch Umwickeln der einzelnen Dragees oder Kapseln mit dünnen Folien aus Papier, Metall oder Plastik erreicht werden. Außerdem können die Kapseln in Röhrchen z.B aus Polystyrol verpackt werden, oder in Folien eingesiegelt werden. Neben Folien aus Zellglas, Aluminium und Papier können auch Kunststofffolien verwendet werden. Im allgemeinen dient PE (Polymerisationsgrad von 3000-4000) als Material für derartige Packmittel. Weitere Möglichkeiten sind Durchdrückpackungen, bei denen auf eine kunststoffolie beispielsweise eine Aluminiumfolie aufgesiegelt ist, oder Schrumpfpackungen. Die Kapseln können z.B auch in Faltschachteln, Kartons, Dosen, oder Kunststofftüten eingebracht werden.
Die Entnahme einzelner erfindungsgemäßer Hohlkugeln, Dragees oder Kapseln kann durch einfaches Herausnehmen mit der Hand erfolgen. Es ist aber auch möglich, die Entnahme der erfindungsgemäßen Kapseln durch ein geeignetes Spendersystem zu erleichtern. Hierzu können z.B. durch Betätigen eines einfachen Mechanismus einzelne Hohlkugeln, Dragees oder Kapseln aus dem Spendersystem freigegeben werden. Beispiel hierfür sind die von der Firma PEZ International AG unter der Marke "PEZ" vertriebenen Spendersysteme für Bonbons und andere Süßwaren.
Vorteilhaft sind aber auch Spendersysteme bei denen auf einer runden Scheibe die Emulsionskapseln schneckenförmig in Vertiefungen eingelagert sind, und durch einen Dosiermechanismus einzeln zu entnehmen sind. Besonders vorteilhaft sind hierbei Ausführungen, die von ihrer äußeren Form her an bekannte Kosmetikprodukte, beispielsweise die bekannte NIVEA-Dose, erinnern, da dies die Gefahr einer Verwechselung mit Lebensmitteln reduziert.

Bei der Anwendung werden einzelne Dragees entnommen und auf der Haut verrieben. Das Produkt wird dabei durch Schmelzen, Scheren oder Lösen der festen oder halbfesten Hüll- oder Füllungsbestandteile niedriger viskos und gut verteilbar und löst sich auf bzw. in der Haut oder dem Haar auf. Dem Fachmanne ist offensichtlich, dass Hülle und/oder Füllung durchaus feste Bestandteile enthalten können, deren Lösen während der Applikation weder möglich noch erwünscht ist, nämlich Feststoffe, wie sie bereits in herkömmlichen Kosmetika eingesetzt werden, ohne dass der Verbraucher bemerkt, dass sie in fester Form vorliegen. Beispiele hierfür sind Füllstoffe, Lichtschutz- und Farbpigmente.

Der Anwender entnimmt eine oder mehrere der Kapseln und verreibt sie auf der Haut, wie er es ansonsten mit Hautcreme aus der Dose oder Tube gewohnt ist. Vorteilhaft ist hier jedoch dass er eine vorportionierte Menge ohne Überreste und Verpackung anwenden kann.

Der Vorteil der erfindungsgemäßen Kapseln ist die bequeme einfache Einmalanwendung für Zwischendurch. Ähnlich dem Schminken oder Einfetten der Lippen ist somit auch ein Eincremen oder die Hautpflege unterwegs möglich. Darüber hinaus kann der Verbraucher einzelne Kapseln auch anderen Verbrauchern anbieten. Dies ist bei herkömmlichen kosmetischen Produkten zwar auch möglich, aber das gemeinsame Benutzen beispielsweise einer Creme aus ein und demselben Tiegel gleicht psychologisch einer körperlichen Berührung. Deshalb existiert hier eine Hemmschwelle, die durch die vorliegende Erfindung beseitigt wird.

Es ist auch möglich, erfindungsgemäße Kapseln mit unterschiedlichen Eigenschaften (z.B. Parfum, Farbe, Hautgefühl, Lichtschutzfaktor, enthaltene Wirkstoffe, und Kombinationen dieser Eigenschaften) in einer Verpackung anzubieten, was bei herkömmlichen Hautpflegeprodukten nicht möglich ist.

Ein weiterer Vorteil ist, dass die Anwendung der erfindungsgemäßen Kapseln einigen Anwendern, vor allem Kindern, mehr Spaß macht als die Anwendung herkömmlicher kosmetischer Produkte. Dies kann es den Eltern erleichtern, ihre Kinder vor schädlichen Umwelteinflüssen wie beispielsweise UV-Strahlung zu schützen.
Dem Fachmann ist bekannt, dass ein großes Problem der Schutz kosmetischer Produkte vor Pilzen, Hefen und Bakterien ist, die während der Anwendung in das Produkt gelangen. Dies geschieht vor allem durch das Anfassen des Produktes durch den Verbraucher während der Entnahme. Es ist offensichtlich, dass die erfindungsgemäßen Kapseln hier einen weiteren Vorteil bieten, da der Verbraucher nur diejenigen Kapseln berührt, die er auch sofort anwendet. Die übrigen Kapseln bleiben so vor mikrobiellem Befall geschützt, weswegen im Vergleich zu herkömmlichen kosmetischen Produkten mit geringeren Gehalten an Konservierungsmitteln gearbeitet werden kann. Da Konservierungsmittel zu den weniger verträglichen kosmetischen Rohstoffen gehören, ist so eine verbesserte Verträglichkeit der Produkte erreichbar, was einen weiteren Vorteil der vorliegenden Erfindung darstellt.

Die kapselförmigen erfindungsgemäßen Zubereitungen können beliebige Formen haben, bevorzugt sind sie jedoch sphärisch mit einem Volumen von 0,1 bis 20 ml.

Die Hülle ist aufgebaut aus Wachsen, Emulgatoren, Polymeren oder Mischungen daraus, Wasser und optional Polyolen, wobei die Polymeren aus der Gruppe Celluloseether. Polyvinylpyrrolidon, Polyacrylate, Polymethacrylate, Polyethylene, Nylon und Eudragit ausgewählt sind. Die Hülle hat ausreichende Schlagstabilität, um mechanische Belastungen während Herstellung und Lagerung auszuhalten, und ist dünn genug, sich bei der Applikation schnell aufzulösen. Bevorzugt beträgt die Hülldicke 0,001 bis 3 mm, insbesondere Dicken im Bereich von 0,01-2 mm.
Auch der Einsatz von Wasser ist in Anteilen von 50% oder 60% problemlos möglich.

Die Hülle wird vorteilhaft aus Wachsen wie Ceresin, Ozokerit, Esterwachse, Glyceridwachse und/oder Fettalkoholen sowie festen Emulgatoren und deren Mischungen aufgebaut. Die Wachse können ihrer Herkunft nach Naturwachse, modifizierte Naturwachse, teilsynthetisch oder vollsynthetisch sein.
Alle Bestandteile werden so ausgewählt, dass sie die geforderte Form- und Temperaturstabilität gewährleisten, die Eintrocknung der Füllung durch Verdunsten verhindern und bei der Applikation schnell schmelzen, aufgrund von Scherkräften völlig oder teilweise flüssig werden oder sich im Füllmaterial auflösen.

Zur Optimierung der elastischen Eigenschaften können Polymere in die Hülle eingearbeitet werden. Geeignete Polymere sind Celluloseether, Polyvinylpyrrolidon, Polyacrylate oder Polymethacrylate, sowie Eudragit.
Das erfindungsgemäße Hüllmaterial setzt sich bevorzugt aus Wachsen zusammen, die aus der Gruppe der
- natürlichen Wachse, besonders bevorzugt Carnaubauwachs, Candelillawachs, Shellackwachs, Beerenwachs (Rhus Verniciflura), hydrierte Pflanzenöle, wie hydriertes Palm- oder Rapsöl, Bienenwachs, Wollwachs (Eucerit)
- Mono-, Di - und Triglyceride aus höheren gesättigten Fettsäuren mit 10 - 30 Kohlenstoffatomen oder Mischungen hieraus, besonders bevorzugt Glyceryltripalmitat (Dynasan 116) und/oder Glycerylstearat, Kahlwachs 6447 (Gemisch aus Fettsäureestern und Kohlenwasserstoffpolymer), Glyceryltribehenat (Syncrowax HRC)
- höheren gesättigten Fettalkohole, besonders bevorzugt solche mit 14 - 30 Kohlenstoffatomen, ganz besonders bevorzugt Stearylalkohol und/oder Behenylalkohol und/oder Cetylalkohol
- synthetischen Ester, bevorzugt C16-36 Alkylhydroxystearoylstearat, Stearylstearat, Cetearylbehenat, C20-40 Alkylstearat, besonders bevorzugt Cetylpalmitat, Methylpalmitat, Methylstearat, Myristylmyristat, Myristyllactat, Cetyllactat, Stearyllactat
- Polymerwachse, bevorzugt Polyethylen, Polypropylen, Polyvinylether, Polydecen besonders bevorzugt Polyvinylstearylether und hydriertes Polydecen,
- Copolymere, besonders bevorzugt solche aus Ethylen- und Vinylacetat sowie aus Polyvinylpyrrolidon und Hexadecen,
- Kohlenwasserstoffe/ Paraffinwachse, besonders bevorzugt Cera Microcristallina, Paraffinwachs, Ceresin, Ozokerit
- Silikonwachse
- chemisch modifizierten Wachse
- beliebigen Mischungen aus Wachsen der genannten Gruppen
gewählt werden.

Vorteilhaft sind Hüllzusammensetzungen, die Rohstoffe und/oder Rohstoffkombinationen mit möglichst scharfen Schmelzpunkten oder engen Schmelzbereichen bei ca. 30°C enthalten. Dies können Einzelstoffe sein wie beispielsweise
- Ester von C12-C24-Fettsäuren mit kurzkettigen Alkoholen (C1-C6),
- Ester kurzkettiger organischer Säuren (C2-C6) mit mittel- oder langkettigen Fettalkoholen (C12-C24)
- Ester der Pyrrolidoncarbonsäure mit mittel- oder langkettigen Fettalkoholen (C12-C24)

Die genannten Säuren oder Alkohole können zusätzliche Gruppen umfassen wie z.B. Alkyl- oder Hydroxygruppen (z.B. Milchsäure).

Darüber hinaus können auch eutektische Gemische mit einer entsprechenden Eutektikumstemperatur verwendet werden, z.B. Mischungen von hydriertem Kokosfett mit Wachsestern (C12-C24 Fettsäure verestert mit C12-C24 Fettalkohol). Die Auswahl geeigneter eutektischer Gemische durch Untersuchung des Schmelzverhaltens von Mischungen (bspw. mittels Differential Scanning Calorimetry DSC) ist dem Fachmann bekannt.

Erfindungsgemäß besonders bevorzugte Wachse zur Herstellung der erfindungsgemäßen Hülle sind Cetylpalmitat, Cetylrizinoleat, Bienewachs, hydrierte Kokosglyceride, Methylpalmitat, Methylstearat, Methylstearat, Ethylstearat, Myristyllactat, Cetyllactat, Stearyllactat, Candelillawachs, Carnaubawachs, Paraffinwachs, Ceresin, Ozokerit, Myristylmyristat, Tripalmitin, Tribehenin, Glycerylpalmitostearat, hydriertes Rapsöl und C15-C40 Alkylstearylstearat.

Im Rahmen der vorliegenden Offenbarung wird als Oberbegriff für Fette, Öle, Wachse und dergleichen gelegentlich der Ausdruck "Lipide" verwendet, wie dem Fachmann durchaus geläufig ist. Auch werden die Begriffe "Ölphase" und "Lipidphase" synonym angewandt.

Wachse im Sinne der vorliegenden Erfindung weisen vorteilhaft einen Schmelzpunkt von 20 bis 90 °C, bevorzugt einen Schmelzpunkt von 25 bis 80 °C und besonders bevorzugt einen Schmelzpunkt von 30 bis 70 °C auf.
Um den Gesamtwachsgehalt der Kapseln zu reduzieren und so ein angenehmeres und ausgewogeneres Hautgefühl zu erreichen, kann es vorteilhaft sein, in die Wachshülle fein dispergierte Wassertröpfchen einzubauen, was beispielsweise im Bereich der Farbkosmetik und Lippenpflege Anwendung findet. Bei Raumtemperatur feste Lippenpflegestifte aus solchen W/O-Dispersionen werden beispielsweise in der Schrift DE 10148313 beschrieben. Diese dort beschriebene Technologie ist hiermit Gegenstand der vorliegenden Erfindung.

Zusätzlich können auch noch andere feste Stoffe in der Hülle enthalten sein, die zu allen Zeitpunkten (Herstellung, Lagerung, Anwendung) in fester Form vorliegen (z.B. anorganische Pigmente, Lipidverdicker auf Aerosil-, Hectorit-, Bentonit- oder allgemein Mineralbasis) oder über breite Schmelzbereiche oberhalb 30°C verfügen (natürliche Fette und Wachse wie Shea- oder Kokosbutter, Carnauba- oder Candelillawachs, gehärtete Fette wie hydriertes Kokosfett, Mikrokristalline Wachse, Ceresine etc.).

Die Füllung kann aus einem wasserhaltigen Medium, beispielsweise einer Emulsion (o/w, w/o, w/o/w), einem Gel, einer Mikroemulsion oder einer Hydrodispersion bestehen. Das Füllmaterial kann aus allen in der Kosmetik bekannten Stoffen und Zubereitungen bestehen, insbesondere O/W- oder W/O/W-Emulsionen in Form von Cremes oder wässrige Gele sind vorteilhaft.

Besonders vorteilhaft und damit erfindungsgemäß bevorzugt besteht die Hülle aus einer W/O-Emulsion und/oder Wachsen und die Füllung aus O/W-Emulsionen, wasserhaltigen Zusammensetzungen, Hydrogelen und/oder Hydrokolloiden. Diese Auswahl vermindert das gegebenenfalls mögliche Vermischen von Hüll- und Füllbestandteilen.

Hülle und Füllung können unabhängig voneinander die üblichen Hilfs- und Zusatzstoffe enthalten, die dem Fachmanne natürlich bekannt sind. Vorteilhaft ist dabei aber, dass die Zusatzstoffe unabhängig voneinander in der Hülle und/oder in der Füllung vorhanden sein können. Beispielsweise können somit Farbstoffe lediglich in der Hülle eingebracht werden ohne die Füllbestandteile zu verändern und dennoch ansprechende ästhetische Effekte erzielt werden. Die erfindungsgemäßen kosmetischen Zubereitungen können daher sowohl in der Hülle als auch in der Füllung kosmetische Hilfsstoffe enthalten, wie sie üblicherweise in Kosmetika verwendet werden, z.B. Konservieungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Geschmacksstoffe, Vergällungsmittel, Parfums, Verdickungsmittel, weichmachende Substanzen, anfeuchtende und/oder feuchthaltende Substanzen, Antioxidantien, UV-Filtersubstanzen, Sensorikadditive, Filmbildner, Tenside, Emulgatoren, Fette, Öle, Wachse, Wirkstoffe oder andere übliche Bestandteile einer kosmetischen Formulierung wie Alkohole, Polyole, stabilisierende Polymere, Schaumstabilisatoren, Elektrolyte, organische Lösungsmittel oder Silikonderivate.

Bevorzugte Emulgatoren sind O/W-Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel

   R-O-(-CH₂-CH₂-O-)ₙ-R',
- der Fettsäureethoxylate der allgemeinen Formel

   R-COO-(-CH₂-CH₂-O-)ₙ -H,
- der veretherten Fettsäureethoxylate der allgemeinen Formel

   R-COO-(-CH₂-CH₂-O-)ₙ -R',
- der veresterten Fettsäureethoxylate der allgemeinen Formel

   R-COO-(-CH₂-CH₂-O-)ₙ -C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH

   nd n eine Zahl von 5 bis 30 darstellen,
- der Polyoxyethylensorbitolfettsäureester,
- der Alkylethersulfate der allgemeinen Formel

   R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel

   R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,

   .

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15),
Polyethylenglycol(16)stearylether (Steareth-16), Polyethylenglycol(17)stearylether (Steareth-17), Polyethylenglycol-(18)stearylether (Steareth-18), Polyethylenglycol(1 9)stearylether (Steareth-19), Polyethylenglycol(20)stearylether (Steareth-20),

Polyethylenglycol(12)isostearylether (Isosteareth-12), Polyethylenglycol(13)isostearyl-ether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19), Polyethylenglycol(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(1 4)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycoi(1 4)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearyl-ether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)-cetylstearylether (Ceteareth-19), Polyethylenglycol(20)cetylstearylether (Ceteareth-20),

Es ist ferner von Vorteil, die Fettsäureethoxylate aus folgender Gruppe zu wählen: Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,

Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlau-reth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesteryl-ether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol-(22)glyceryllaurat, Polyethylenglycol(23)glyceryllaurat, Polyethylenglycol(6)glycerylcap-rat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitan-monoisostearat, Polyethylenglycol(20)sorbitanmonopalmitat, Polyethylenglycol(20)sorbi-tanmonooleat zu wählen.

Besonders bevorzugte O/W-Emulgatoren sind Triceteareth-4-Phosphat, Polyglyceryl-3-Methylglucosedistearat, Polyethylenglycol-40-Stearat, Glycerylstearatcitrat, Ceteareth-20, Ceteareth-2, Cethyl-Dimethicone-Copolyol; Wollwachsalkohol, Methylglucosesesquistearat, PEG-PPG-Blockpolymere (Pluronics F68/127), Cetearylglucosid, Stearinsäure.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter oder unverzweigter Alkancarbonsäuren einer Kettenlänge von 8 bis 24, insbesondere 12 bis 20 Kohlenstoffatomen, Diglycerinester gesättigter und/oder ungesättigter, verzweigter oder unverzweigter Alkancarbonsäuren einer Kettenlänge von 8 bis 24, insbesondere 12 bis 20 Kohlenstoffatomen, Monoglycerinether gesättigter und/oder ungesättigter, verzweigter oder unverzweigter Alkohole einer Kettenlänge von 8 bis 24, insbesondere 12 bis 20 Kohlenstoffatomen, Diglycerinether gesättigter und/oder ungesättigter, verzweigter oder unverzweigter Alkohole einer Kettenlänge von 8 bis 24, insbesondere 12 bis 20 Kohlenstoffatomen, Polypropylenglykolester gesättigter und/oder ungesättigter, verzweigter oder unverzweigter Alkancarbonsäuren einer Kettenlänge von 8 bis 24, insbesondere 12 bis 20 Kohlenstoffatomen, Polyglycerylester gesättigter und/oder ungesättigter, verzweigter oder unverzweigter Alkancarbonsäuren einer Kettenlänge von 8 bis 24, insbesondere 12 bis 20 Kohlenstoffatomen, Sorbitanester gesättigter und/oder ungesättigter, verzweigter oder unverzweigter Alkancarbonsäuren einer Kettenlänge von 8 bis 24, insbesondere 12 bis 20 Kohlenstoffatomen Lanolinalkohol

Bevorzugte W/O-Emulgatoren sind verzweigte oder unverzweigte, gesättigte oder ungesättigte Fettsäuren mit 12 bis 26 Kohlenstoffatomen, Polyglyceryl-3 Diisostearat, Polyglyceryl-4 Isostearat, Polyglyceryl-2 Dipolyhydroxystearat, Cetyl PEG/PPG-10-1-Dimethicone, PEG-30 Dipolyhydroxystearat, PEG-40 Sorbitanperisostearat, Cetyldimethiconecopolyol, PEG-7 Hydrogenated Castor Oil, PEG 45/Dodecylglycolcopolymer, PEG 22/Dodeceylglycolcopolymer, Pentaerythritylisostearat, Isostearyldiglycerylsuccinat, Sorbitanisostearat, Polyglyceryl-2 Sesquiisostearat, Glycerylisostearat, Sorbitanstearat, Glycerylstearat, PEG-25 Hydrogenated Castor Oil, PEG-40 Sorbitanperoleat, Sorbitanoleat, PEG-40 Sorbitanperisostearat, Polyglyceryl-3 Oleat, Polyglyceryl-2 Sesquioleat und Polyglyceryl-4 Isostearat.

Besonders bevorzugte W/O-Emulgatoren sind Poylethylenglycol-45/Dodecylglycolcopolymer, Polyglyceryl-3-Diisostearat, PEG-30 Dipolyhydroxystearate, Sorbitanisostearate, Sorbitan Stearate, Glyceryl Isostearate, Glyceryl Stearate und Sorbitan Oleate.

Es ist auch möglich, auf das Herabsetzen der Grenzflächenenergie durch Emulgatoren oder Tenside zu verzichten und statt dessen die Grenzfläche durch Anlagerung von in beiden Phasen unlöslichen Partikeln zu stabilisieren. Hierzu können natürliche oder künstliche Polymere (Polyethylen, Nylon, Stärke und ihre Derivate) oder anorganische Partikel (TiO₂, Al₂O₃, BaSO₄, BN, Silikate, Alumosilikate) verwendet werden.

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.
Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC erhältlich ist.

Es ist ferner bevorzugt, die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* erhältliche), Hexadecylbenzoat und Butyloctylbenzoat und Gemische davon (*HaIIstar AB*) und/oder Diethylhexylnaphthalat (*Hallbrite TQ oder Corapan TQ von H*&*R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 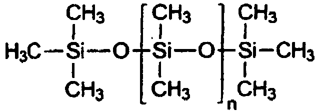 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw.
Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphe-nylsiloxan).

Die hier vortteilhaft beschriebenen Ölphasen können sowohl in der Hülle als auch in der Füllung vorhanden sein.

Es ist ebenfalls vorteilhaft, den Kapseln, der Hülle und/oder der Füllung im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.
Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Retinoide wie zum Beispiel Retinol, Retinal und/oder Retinsäure und die jeweiligen Ester, α-Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl-und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Äpfelsäure), Huminsäure, Gallensäure, Gallenextrakte, Bilirubin, Biliverdin, EDTA, IDS, EGTA und deren Derivate, ungesättigte Fettsäuren und deren Derivate (z.B. γ-Linolensäure, Linolsäure, Ölsäure), Folsäure und deren Derivate, - 2-Aminopropionsäurediessig-säure, Flavonoide, Polyphenole, Catechine, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.
Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.
Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Weiterhin können der erfindungsgemäßen Zubereitung UV-Filtersubstanzen zugesetzt werden. Es ist damit bevorzugt die erfindungsgemäßen Zubereitungen als Lichtschutzformulierungen anzuwenden.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure (2-ethylhexyl)ester (2-Ethylhexyl-4-methoxycinnamat, INCI: Octyl Methoxycinnamate) und 4-Methoxyzimtsäureisopentylester (Isopentyl-4-methoxy-cinnamat, INCI: Isoamyl p-Methoxycinnamate).

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).
Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.
Die Pigmente können erfindungsgemäß vorteilhaft oberflächlichbehandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.
Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.
Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.
Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Ebenfalls geeignete UV-A Filtersubstanzen sind Hydroxybenzophenone. Diese zeichnen sich durch die folgende Strukturformel aus: 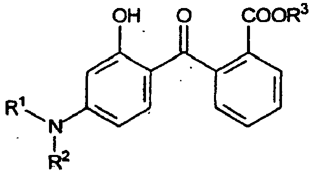 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 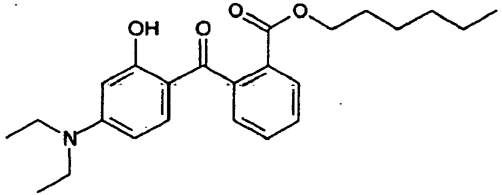 und unter dem Uvinul A Plus bei der Fa. BASF erhältlich ist.
Die Gesamtmenge an einem oder mehreren Hydroxybenzophenonen in den fertigen kosmetischen oder dermatologischen Zubereitungen wird vorteilhaft aus dem Bereich 0,01 Gew.-% bis 20 Gew.-%, vorzugsweise von 0,1 bis 10 Gew.-% gewählt, jeweils bezogen auf das Gesamtgewicht der Zubereitungen.
Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 oder unter Neo Heliopan Hydro bei erhältlich ist;
- 1,4-di(2-oxo-10-Sulfo-3-bornylidenmethyl)-Benzol (auch: 3,3'-(1,4-Phenylendimethy-lene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX erhältlich;
- Sulfonsäure-Derivate des 3-Benzylidencamphers, wie z. B. 4-(2-Oxo-3-bornyliden-methyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S erhältlich ist;
- Diethylhexylbutylamidotriazon (INCI: Diethylhexylbutamidotriazone), welches unter der Handelsbezeichnung UVASORB HEB erhältlich ist;
- 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M erhältlich ist.
Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]di-siloxanyl]propyl]-phenol (CAS-Nr.: 155633-54-8) mit der INCI-Bezeichnung Drometrizole Trisiloxane, welches unter der Handelsbezeichnung Mexoryl® XL erhältlich ist.
Die weiteren UV-Filtersubstanzen können öllöslich oder wasserlöslich sein. Vorteilhafte öllösliche UV-B- und/oder Breitband-Filtersubstanzen im Sinne der vorliegenden Erfindung sind z. B.:
▪ 3-Benzylidencampher-Derivate, vorzugsweise 3-(4-Methylbenzyliden)campher, 3-Benzylidencampher;
▪ 4-Aminobenzoesäure-Derivate, vorzugsweise 4-(Dimethylamino)-benzoesäure(2-ethylhexyl)ester, 4-(Dimethylamino)benzoesäureamylester;
▪ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
▪ sowie an Polymere gebundene UV-Filter.
▪ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX erhältlich ist.

Vorteilhafte wasserlösliche Filtersubstanzen sind z. B.:
Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.
Eine weiterere erfindungsgemäß vorteilhaft zu verwendende Lichtschutzfiltersubstanz ist das Ethylhexyl-2-cyano-3,3-diphenylacrylat (Octocrylen), welches unter der Bezeichnung Uvinul^{®} N 539 erhältlich ist.
Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A-und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.
Die Liste der genannten UV-Filter, die im Sinne der vorliegenden Erfindung eingesetzt werden können, soll selbstverständlich nicht limitierend sein.
Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Erfindungsgemäße Zubereitungen enthalten insbesondere vorteilhaft ein oder mehrere Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Diese Hydrocolloide können vorteilhaft aus der vorgenannten Gruppe gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Vorteilhaft sind außerdem Taurate, z.B. Ammonium Acryloyldimethyltaurat / VP Copolymer.

Unter den Polyacrylaten befinden sich z.B. Carbopol Typen der Firma Goodrich (Carbopol 980, 981, 1382, 5984, 2984, ETD 2001, ETD 2020, ETD 2050, Ultrez-1 0 oder Pemulen TR1 & TR2).

Unter den Polymeren befinden sich z.B. Polyacrylamide (Seppigel 305), Polyvinylalkohole, PVP, PVP / VA Copolymere, Polyglycole.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder - monoethylether, Ethylhexyloglycerin, Methylpropandiol und analoge Produkte, Polymere, Schaumstabilisatoren, Elektrolyte wie beispielsweise Natriumchlorid oder Magnesiumsulfat sowie insbesondere ein oder mehrere Verdickungsmittel, welches oder welche vorteilhaft gewählt werden können aus der Gruppe Siliziumdioxid, Alumosilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon, früher Goodrich], beispielsweise Carbopole der Typen 980, 981, 1382, 2984, 5984, ETD 2020, ETD 2050, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 erhältlich). Die Repellentien können sowohl einzeln als auch in Kombination eingesetzt werden.

Als Feuchthaltemittel und/oder hautbefeuchtende Stoffe werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Panthenol, Fucogel, Glycine Soja, Ethylhexyloxyglycerin, Pyrrolidoncarbonsäureund ihre Derivate sowie Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke-Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Neben der zuvor beschriebenen Anwendung der Kapseln als Sonnenschutz, Haut- und Lippenpflege, Selbstbräunung oder Insektenrepellent, wobei diesbezügliche Inhaltstoffe individuell gewählt werden können, ist die erfindungsgemäße Kapsel auch als Reinigungskapsel anwendbar.

In der Füllung finden sich dann Tenside oder waschaktive Substanzen, die beim Verreiben auf der Haut frei gesetzt werden. Die Einarbeitung als Füllbestandteil erfolgt dabei ohne jegliche Komplikationen.
Ein Problem bei der Hand- oder Gesichtsreinigung bestand darin, dass die Menge an Waschmitteln schwer zu dosieren war. Durch die tensidhaltigen Kapseln ist dem Anwender eine Möglichkeit gegeben, immer gleiche Mengen an Waschmittel einzusetzen. Dadurch das die Kapseln bei der Benutzung keine Rückstände hinterlassen, fällt keine zusätzliche Verpackung an.
Vorteilhaft ist insbesondere bei Bereitstellung der Kapsel mit Tensiden die Kombination der waschaktiven Substanzen in der Füllung mit dem Wachs in der Hülle. Somit hat man beim Auflösen der Kapseln beim Verreiben oder unter warmen Wasser eine Formulierung, die schäumt und durch den Wachsanteil in der Lage ist auch wasserfeste Rückstände, wie beispielsweise Make-up, zu entfernen. Auf Reisen kann somit gezielt und zwischendurch das Gesicht und Haut gereinigt werden ohne das aufwendige Reinigungsmittel mitgenommen werden müssen.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat-oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂ H₂COOH X⁻ | (bei pH=2) | X⁻ = beliebiges Anion, z.B. Cl⁻ |
| RNH₂⁺CH₂CH₂COO | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine Ionen.
1. A. Anionische Tenside
   Vorteilhaft zu verwendende anionische Tenside sind
   Acylaminosäuren (und deren Salze), wie
   1. 1. Acylglutamate, beispielsweise Natriumacylglutamat, Di-TEApalmitoylaspartat und Natrium Caprylic/ Capric Glutamat,
   2. 2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
   3. 3. Sarcosinate, beispielsweise Myristoyl Sarcosin, TEA-lauroyl Sarcosinat, Natriumlauroylsarcosinat und Natriumcocoylsarkosinat,
   4. 4. Taurate, beispielsweise Natriumlauroyltaurat und Natriummethylcocoyltaurat,
   5. 5. Acyllactylate, Lauroyllactylat, Caproyllactylat
   6. 6. Alaninate
   Carbonsäuren und Derivate, wie
   7. 1. Carbonsäuren, beispielsweise Laurinsäure, Aluminiumstearat, Magnesiumalkanolat und Zinkundecylenat,
   8. 2. Ester-Carbonsäuren, beispielsweise Calciumstearoyllactylat, Laureth-6-Citrat und Natrium PEG-4-Lauramidcarboxylat,
   9. 3. Ether-Carbonsäuren, beispielsweise Natriumlaureth-13-Carboxylat und Natrium PEG-6-Cocamide Carboxylat,
   Phosphorsäureester und Salze, wie beispielsweise DEA-Oleth-10-Phosphat und Dilaureth-4 Phosphat,
   Sulfonsäuren und Salze, wie
   10.1. Acyl-isethionate, z.B. Natrium-/ Ammoniumcocoyl-isethionat,
   11.2. Alkylarylsulfonate,
   12.3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
   13.4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
   sowie
   Schwefelsäureester, wie
   14.1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
   15.2. Alkylsulfate, beispielsweise Natrium-, Ammonium- und TEA-Laurylsulfat.
2. B. Kationische Tenside
   Vorteilhaft zu verwendende kationische Tenside sind
   1. 1. Alkylamine,
   2. 2. Alkylimidazole,
   3. 3. Ethoxylierte Amine und
   4. 4. Quaternäre Tenside.
   5. 5. Esterquats
   Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide,
   Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.
3. C. Amphotere Tenside
   Vorteilhaft zu verwendende amphotere Tenside sind
   1. 1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
   2. 2. N-Alkylaminosäuren, beispielsweise Aminopropylalkylglutamid, Alkylaminopropionsäure, Natriumalkylimidodipropionat und Lauroamphocarboxyglycinat.
4. D. Nicht-ionische Tenside
   Vorteilhaft zu verwendende nicht-ionische Tenside sind
   1. 1. Alkohole,
   2. 2. Alkanolamide, wie Cocamide MEA/ DEA/ MIPA,
   3. 3. Aminoxide, wie Cocoamidopropylaminoxid,
   4. 4. Ester, die durch Veresterung von Carbonsäuren mit Ethylenoxid, Glycerin, Sorbitan oder anderen Alkoholen entstehen,
   5. 5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
   6. 6. Sucroseester, -Ether
   7. 7. Polyglycerinester, Diglycerinester, Monoglycerinester
   8. 8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Zur Anwendung werden die kapselförmigen Zubereitungen erfindungsgemäß in der für Kosmetika üblichen Weise auf die Haut und/oder die Haare aufgebracht und verrieben bzw. verteilt.

Der erfindungswesentliche Vorteil ist die erstmalige Bereitstellung einzelner handhabbarer kosmetischer Präparate in Kapselform, die dem Verbraucher eine vereinfachte Entnahme, verbesserte Hygiene beim Teilen des Produktes mit anderen sowie eine neue Applikationsform bieten.

Die erfindungsgemäßen kosmetischen und/oder dermatologischen Zubereitungen können wie übliche kosmetische und/oder dermatologische Zubereitungen zusammengesetzt sein und dem kosmetischen und/oder dermatologischen Lichtschutz, zur Änderung oder Beeinflussung bestimmter Hautzustände, ferner zur Behandlung, der Pflege und der Reinigung der Haut und/oder der Haare und als Schminkprodukt in der dekorativen Kosmetik dienen.

Entsprechend können kosmetische und/oder topische dermatologische Zusammensetzungen im Sinne der vorliegenden Erfindung, je nach ihrem Aufbau, beispielsweise verwendet werden als Hautpflegeprodukt, Hautschutzprodukt, Reinigungsprodukt, Sonnenschutzprodukt, Haarkur, Körperreinigungsprodukt, für die Tages- oder Nachtpflege, die Pflege bestimmter Hautareale wie Hände, Gesicht, Füße usw.

Auch ist die Verwendung der erfindungsgemäßen kapselförmigen kosmetischen Zubereitungen zur Prophylaxe und Behandlung der Symptome von Altershaut, zur Verhinderung und Verminderung der Entstehung und Ausbreitung von Fältchen und Falten sowie zur Behandlung und Pflege gealterter Haut erfindungsgemäß. So lässt sich vorteilhaft eine einzelne Kapsel, enthaltend Ubichinon, Ubichinol, Retinol und Derivate, Dehydroepiandrosteron (DHEA), Isoflavonoide (insbes. Genistein, Daidzein), Creatin, Phytoöstrogene, Östrogen, Östradiol und Derivate, Niacinamid, Polyphenole (AGR) oder eine andere, gegen Falten wirksame Substanz auf die Gesichtshaut auftragen und verteilen.

Weiterhin ist die Verwendung der erfindungsgemäßen kapselförmigen kosmetischen Zubereitungen zur Prophylaxe und Behandlung der Symptome trockener Haut bevorzugt. Geeignete Wirkstoffe für diesen Einsatzzweck sind: natürliche Öle (Sonnenblumen-, Nachtkerzensamen-, Jojoba-, Macadamiaöl, Rhizinusöl), Ceramide, insbes. Ceramid I, III und VI, Cholesterin, Phytosterole, Fettsäuren mit einer Kettenlänge von C16-26, Carnitin und seine Derivate, Harnstoff, Polyole wie Glycerin, Butylenglykol, Propylenglykol und Dipropylenglykol, Pseudoceramide; Elektrolyte wie Natriumchlorid und Taurin, Fettalkohole sowie Wachse.

Außerdem ist die Verwendung der erfindungsgemäßen kapselförmigen kosmetischen Zubereitungen zur Prophylaxe und Behandlung der Symptome der sensiblen bzw. entzündeten Haut vorteilhaft. Bevorzugte Wirkstoffe für diesen Einsatzzweck sind: Inhaltsstoffe der Mariendistel, insbesondere Silymarin, Hamamelisextrakt, Kamillenextrakt, Inhaltsstoffe der Süßholzpflanze (Glycerrhizinsäure, Licochalcone A & B), Allantoin, Acetylsalicylsäure, Diclofenac, Pentacyclische Triterpene (Sericoside, Ursolsäure) und Panthenol.

Außerdem ist die Verwendung der erfindungsgemäßen kapselförmigen kosmetischen Zubereitungen zur Prophylaxe und Behandlung der Symptome der fehlpigmentierten Haut vorteilhaft. Bevorzugte Wirkstoffe für diesen Einsatzzweck sind: Tyrosinaseinhibitoren, Hydrochinonderivate, Dioic Acid, Liponsäure und ihre Derivate sowie Kojisäure.

Es ist gegebenenfalls möglich und vorteilhaft, die erfindungsgemäßen Zusammensetzungen als Grundlage für pharmazeutische Formulierungen zu verwenden. Weiterhin ist die Verwendung der erfindungsgemäßen kapselförmigen kosmetischen Zubereitungen zur Prophylaxe und Behandlung der Symptome kranker Haut bevorzugt. Relevante aber nicht ausschließliche krankhafte Hautzustände sind Psoriasis, Akne, Neurodermitis und andere atopische Erkrankungen wie atopische Dermatitis, Hautkrebs, Herpes, Mykosen, Ichthyosis, Pityriasis, Seborrhöe, Pellagra, Kontaktekzeme und Allergien. Geeignete Wirkstoffe für solche Einsatzzwecke sind Antibiotika wie Fusidinsäure, Erythromycin, Sulfadiazin, Clindamycin, Tetracycline, Tyrothricin Aminoglycoside, Bacitracin, Chloramphenicol, Virustatika (z.B. Aciclovir, Idoxuridin, Penciclovir), Antimykotika (z.B. Nystatin, Amphotericin, Clotrimazol, Econazol, Ketoconazol,Naftifin, Terbinafin), Allethrin, Zytostatika (5-Fluorouracil), Antiphlogistica (Hydrocortison, Bethamethason; Prednisolon, Triamcinolonacetonid, Dexamethason, Diclofenac, Bufexamac), Immunosuppressiva (Cyclosporin A, Interferon-beta), Antipsoriatica (Dithranol, Psoralen, Tazaroten), Aknemittel (Retinsäure, Isotretinoin, Benzoylperoxid, Adapalen); Capsaicin, Azelainsäure, Keratolytika (Salicylsäure, Milchsäure), Antihistaminika (Azelastin, Levocabastin, Dinatrium-cromoglycin); Antipsoriatika (Dithranol, Calcitriol, Psoralen) und Vitamine (besonders die A-, B- und C-Vitamine).

Eine mögliche und erfindungsgemäß besonders vorteilhafte Anwendung ist es, Kapseln mit unterschiedlichen Verwendungszwecken in einer Packung anzubieten, beispielsweise solche zur Tages- und Nachtpflege, solche mit unterschiedlichen Farben, Düften, unterschiedlich starken Lichtschutzfaktoren oder unterschiedlichen Wirkstoffen. Es ist bei einer solchen Verwendung besonders vorteilhaft, die Kapseln unterschiedlicher Zusammensetzung durch unterschiedliche Form- und/oder Farbgebung für den Anwender unterscheidbar zu machen.

Nicht zuletzt ist die Verwendung der erfindungsgemäßen kosmetischen und/oder dermatologischen Zubereitungen zur Prophylaxe, Behandlung und Reinigung fettiger Haut sowie zur Prophylaxe und Behandlung von unreiner Haut sowie von Cellulite erfindungsgemäß.

Zur Herstellung der erfindungsgemäßen kapselförmigen Zubereitungen werden die Hülle und die Füllung zunächst getrennt voneinander hergestellt. Das Überziehen der Füllung mit dem Füllmaterial kann, unabhängig von der Zusammensetzung des Füllmaterials oder der Hülle, auf verschiedene Arten erfolgen.
Beispielsweise kann das Füllmaterial eingefroren und dann in aufgeschmolzenes Hüllmaterial, getaucht werden, wodurch sich auf dem Füllmaterial eine feste, geschlossene Hülle bildet.,
Es können aber auch aus geschmolzenem Hüllmaterial Hohlkugeln gegossen werden, die durch ein Loch in der Kugelwand ggf. mit Füllmaterial befüllt werden. Danach wird das Loch durch einen Stopfen aus Hüllmaterial verschlossen. Es ist auch möglich, zunächst halbe Hohlkugeln zu gießen, diese ggf. zu befüllen, zur Deckung zu bringen und abschließend durch thermische Behandlung miteinander zu verschmelzen. Darüber hinaus können zwei solcher Halbkugeln hergestellt werden, wobei eine oder beide ein Loch zu späteren Befüllung aufweisen, dann zu einer Hohlkugel verschweißt und abschließend durch das Füll-Loch befüllt werden, dass dann wie oben beschrieben verschlossen wird.

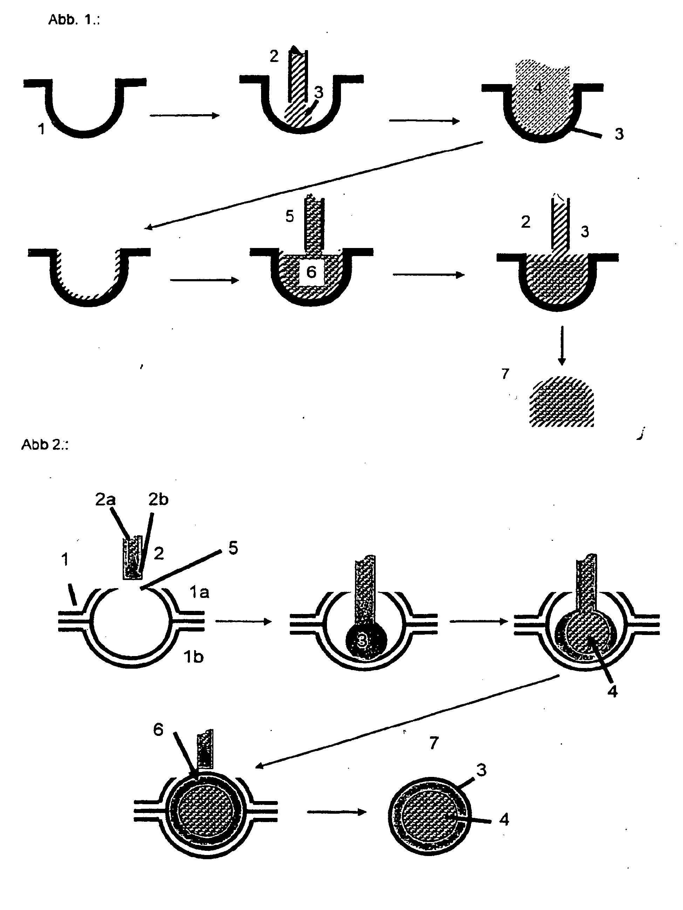

Ein besonders bevorzugtes und erfindungsgemäßes Verfahren zur Herstellung der Kapseln bzw. deren Hülle verläuft nach dem Prinzip des frozen-cone bzw. cold-stamp Verfahrens wie sie beispielsweise in DE 19852262 oder DE 9321186 beschrieben sind. Diese Verfahren aus der Lebensmitteltechnologie sind erfindungsgemäß auf die Herstellung kosmetischer Präparate angepasst worden.
Das frozen-cone-Verfahren wird in der beigefügten Abbildung 1 verdeutlicht. In eine Form 1 wird durch eine Düse 2 zunächst eine definierte Menge Hüllmasse 3 gefüllt. Durch Einpressen eines gekühlten Formkörpers 4 (engl. "frozen cone" oder "cold stamp") wird die eingebrachte Hüllmasse geformt und gleichzeitig so gekühlt, dass sie bis zum Einfüllen der Füllmasse 6 durch eine weitere Düse 5 ihre Form nicht oder nur unwesentlich verändert. Nach dem Einbringen der Füllmasse wird durch die Düse 2a weitere Hüllmasse 3a aufgebracht. Die Düsen 2 und 2a und die Massen 3 und 3a können, müssen aber nicht identisch sein. Es ist vorteilhaft, die Massen 3 und 3a identisch zu wählen. Schließlich wird die fertige Emulsionskapsel 7 entformt. Alternativ können nach dem Einbringen der Füllung zwei noch unverschlossene Halbkapseln aufeinander zur Deckung gebracht und ihre Hüllen miteinander verschmolzen werden.

Mit Hilfe dieses Verfahrens sind die Hülldicken mit hoher Reproduzierbarkeit im Bereich von 0,001 bis 3 mm variabel einstellbar. Gegenüber dem Stand der Technik sind damit erstmalig extrem dünnwandige Kapselpräparate herstellbar.

Ein weiterer Vorteil des Verfahrens ist, dass die nachträglich hinzuzufügende Füllung nicht erwärmt zu werden braucht. Dies ist besonders vorteilhaft, wenn die Füllung temperaturempfindliche Stoffe enthält, die bei erhöhten Temperaturen z.B. oxidativ (Vitamine A, B, C und E oder ihre Derivate wie Acetate, Phosphate oder Palmitate, Provitamin B5) oder hydrolytisch (Acetylcarnitin, Parabene) abgebaut werden. Natürlich kann durch die kalte Verarbeitung der Füllung auch Energie gespart werden, was die Herstellungskosten senkt und die Umwelt weniger belastet.

Um die erfindungsgemäßen Kapsel in verschiedensten Formen, Größe und Dicke der Hülle bereit stellen zu können, hat sich das ebenfalls aus dem Lebensmittelsektor bekannte one-shot-Verfahren bewährt. Auch dieses Verfahren wurde erfindungsgemäß für die Herstellung der erfindungsgemäßen kosmetischen Kapseln angepasst.
Dazu wird aus einer oberen und einer unteren Halbform (1a und b) eine Form 1 gebildet (vgl. Abbildung 2), wobei die obere Halbform 1 a eine Öffnung 5 hat. Durch diese wird eine Doppeldüse 2, bei der ein innerer Kanal 2a konzentrisch in einem äußeren Kanal 2b angeordnet ist, in die Form 1 gefahren. Zunächst wird durch den äußeren Kanal etwas Hüllmasse 3 in die Form gefördert, sodass sich ein geschlossener Boden aus Hüllmasse bildet. Kurz darauf setzt auch die Förderung der Füllung 4 durch den inneren Kanal 2a ein. Während des Förderns der beiden Massen bewegt sich dabei die Düse 2 nach oben aus der Form 1 heraus. Abschließend wird die Förderung der Füllung wieder kurz ausgesetzt, damit auch ein geschlossener Deckel 6 entstehen kann. Durch anschließendes Trennen der beiden Halbformen 1 a und b voneinander kann die fertige Emulsionskapsel 7 entformt werden.

Alternativ kann auch auf die obere Halbform 1 a verzichtet werden, so dass die Öffnung 5 von der Öffnung bzw. deren offenen Seite der unteren Halbform 1 b direkt gebildet wird.
In die untere Halbform 1 b werden die Massen 3 und 4 durch die Doppeldüse 2 direkt gefüllt, ähnlich dem Verfahren nach Abbildung 1.
So lassen sich zwar Kapseln aber nicht in Kugelform herstellen, sondern nur Körper mit einer planen Grundfläche (z.B. wie aus dem Lebensmittelsektor bekannte Schokoladen Praline Toffee-Fee oder Mozartkugeln). Aber auch diese Formen sind damit erfindungsgemäß.

Für das "one-shot"- äquivalente erfindungsgemäße Verfahren ist eine möglichst geringe Zügigkeit der Füllmasse notwendig. Unter Zügigkeit versteht man die Eigenschaft, bei der Entnahme eines Teils einer flüssigen oder halbfesten Zubereitung nicht sofort abzureißen, sondern Fäden zwischen dem entnommenen und dem unberührten Teil der Zubereitung zu ziehen. So ist beispielsweise geschmolzener Käse extrem zügig, reines Wasser hingegen ist nicht zügig, zieht also keine Fäden. Zieht nun die Füllmasse Fäden, so reißt sie auch nicht sauber ab, wenn ihre Förderung aufhört. Wegen der entstehenden Fäden kann sich kein vollständig geschlossener Deckel aus Hüllmaterial bilden, wodurch die Emulsionskapsel in ihren mechanischen (Formstabilität, Härte, Elastizität) und anderen Eigenschaften (Wasserverlust, Auslaufen der Füllmasse) nachteilig beeinflusst wird.

Kosmetische Rohstoffe, wie beispielsweise Polyole (besonders Glycerin, Propylenglykol, Butylenglykol, Polyethylenglykol, Pentandiole, Hexandiole, Octandiole) oder Hydrokolloide (besonders Polysaccharide wie Stärken und Stärkederivate, Mannane, Glucane, Xanthan Gum, Guar Gum, gummi arabicum, sowie Polymere oder Co-Polymere der Acrylsäure oder ihrer Ester), können die Zügigkeit der Füllung verstärken und sich so im Herstellverfahren negativ bemerkbar machen. Als Bestandteil der Füllung führt dies beim Befüllen der Hülle zum Fäden ziehen. Die Hülle kann dann nur noch mit Problemen verschlossen werden. Dieses Problem der Füllung kann überraschend durch eine Modifizierung der Hülle gelöst werden, indem der Hülle oberflächenaktive Substanzen, bevorzugt W/O-Emulgatoren zugesetzt werden. Bevorzugte W/O-Emulgatoren sind verzweigte oder unverzweigte, gesättigte oder ungesättigte Fettsäuren mit 12 bis 26 Kohlenstoffatomen, Polyglyceryl-3 Diisostearat, Polyglyceryl-4 Isostearat, Polyglyceryl-2 Dipolyhydroxystearat, Cetyl PEG/PPG-10-1- Dimethicone, PEG-30 Dipolyhydroxystearat, PEG-40 Sorbitanperisostearat, Cetyldimethiconecopolyol, PEG-7 Hydrogenated Castor Oil, PEG 45/Dodecylglycolcopolymer, PEG 22/Dodeceylglycolcopolymer, Pentaerythritylisostearat, Isostearyldiglycerylsuccinat, Sorbitanisostearat, Polyglyceryl-2 Sesquiisostearat, Glycerylisostearat, Sorbitanstearat, Glycerylstearat, PEG-25 Hydrogenated Castor Oil, PEG-40 Sorbitanperoleat, Sorbitanoleat, PEG-40 Sorbitanperisostearat, Polyglyceryl-3 Oleat, Polyglyceryl-2 Sesquioleat und Polyglyceryl-4. Isostearat. Darüber hinaus können auch Wachse mit Gehalten an oberflächenaktiven Substanzen verwendet werden, vor allem solche mit Gehalten an freien Fettsäuren und/oder Fettsäure-Mono-oder -diglyceriden (z.B. Bienenwachse). Sie erleichtern somit wesentlich das Vergießen von Füllungen, die Glycerin, Hydrokolloide und/oder die zuvor beschriebenen Stoffe enthalten. Der sich sonst bildende Faden wird durch den Zusatz der oberflächenaktiven Substanzen durch die Hülle durchtrennt. Der Zusatz gewährleistet somit ein problemloses Verschließen der Hülle.
Das Problem der Füllung (Fäden ziehen) wird erfindungsgemäß durch Hüllbestandteile gelöst.
Das frozen-cone oder cold-stamp-Verfahren zeichnet sich durch seine hervorragende Genauigkeit und Reproduzierbarkeit in der Dicke und Einheitlichkeit der Hülle aus. Es ist besonders gut geeignet, um sehr dünne Hüllen zu gießen (<1 mm).
Der Vorteil des one-shot-Verfahrens liegt in seiner außergewöhnlichen Flexibilität: Durch Einstellen einiger weniger maschineller Parameter lassen sich mit identischen Düsen, Maschinen und Formen Kapseln mit unterschiedlich dicken Hüllen herstellen. Für eine veränderte Form der Kapsel (bspw. oval statt rund) kann dieselbe Maschine verwendet werden, nur die Halbformen müssen ausgetauscht werden.

Die Formen, in die bei beiden Verfahren gegossen wird, können aus unterschiedlichen Materialien hergestellt werden, besonders bevorzugt thermoplastischen Kunststoffen wie Polyolefinen, Vinylpolymeren, Polyamiden, Polyestern, Polyacetalen und Polycarbonaten hergestellt werden. Vorteilhaft werden sie als Einmal-Formen hergestellt (Blister), die zur Verpackung der fertigen Kapsel verwendet werden können.

Beide zuvor erläuterten erfindungsgemäßen Verfahren ermöglichen eine industrielle, maschinengängige Herstellung der kapselförmigen kosmetischen Zubereitungen, so dass die Produktionskosten gesenkt werden und damit auch preiswerte kapselförmige Kosmetika angeboten werden können.

Die erfindungsgemäße Zubereitung weist zudem verbesserte sensorische Eigenschaften auf, die mit wachshaltigen Zubereitungen aus dem Stand der Technik nicht zu erwarten sind. Es wurde eine verbesserte Verteilbarkeit, Einziehungsvermögen, Konsistenz, Hautglättung und verminderte Klebrigkeit festgestellt. Bezüglich geeigneter Methoden zur Bestimmung dieser Parameter kann auf das Wissen des Fachmannes verwiesen werden.

Erstaunlich ist insbesondere, dass die erfindungsgemäße Kapsel ohne Füllung, als Hohlkugel, ebenso als eigenständiges Kosmetikum angewendet werden kann. Die für den kosmetischen oder dermatologischen Zweck enthaltenden Stoffe befinden sich dann erfindungsgemäß alle in der Kapselhülle.

Die Größenangaben wie beispielsweise der Durchmesser der Kapseln sind zu verstehen als Durchmesser in Richtung der Längsausdehnung der Kapselteilchen.

Nachfolgende Beispiele erläutern die erfindungsgemäßen kapselförmigen Zubereitungen. Die Prozentangaben beziehen sich soweit nichts anderes angegeben auf die Gesamtmasse der Zubereitungen.

### Beispiele:

### A. Beispiele für die Zusammensetzung der Füllung (Angaben in Massenprozent bezogen auf das Gesamtgewicht der Füllung)

### Beispiele 1-5: O/W-Cremes

| **Beispiel** | **1** | **2** | **3** | **4** | **5** |
|---|---|---|---|---|---|
| Glycerylstearatcitrat | 2 | | | | |
| Glycerylstearat, selbstemulgierend | | 5 | 3 | 1 | 2 |
| PEG-40-Stearat | | | 1 | | 1 |
| Stearinsäure | | | | 4 | |
| Myristylmyristat | 1 | | | | 1 |
| Behenylalkohol | | | | 1 | |
| Stearylalkohol | 2 | 1 | | | |
| Cetearylalkohol | | | | | 2 |
| Cetylalkohol | 1 | | 3 | 3 | |
| Hydrierte Kokosfettglyceride (Hydrogenated Coco Glycerides) | 2 | | | | |
| Sheabutter | | 1 | | | 2 |
| C12-15 Alkylbenzoat | | 3 | 2 | | 3 |
| Butylenglycol Dicaprylat/Dicaprat | 1 | | | 1 | |
| Caprylsäure/Caprinsäure Triglycerid | | 1 | 2 | 2 | 2 |
| Ethylhexylkokosfettsäureester | 3 | | | | 1 |
| Octyldodecanol | | | 1 | | |
| Jojobaöl | | | | 1 | |
| Mineralöl | | 1 | | | |
| Vaseline | 1 | | 1 | | 2 |
| | | | | | |
| Cyclomethicon | 3 | 2 | 4 | 3 | 5 |
| Dimethicon | | | 1 | 1 | |
| Dicaprylylether | 1 | 3 | 2 | | |
| Dicarprylylcarbonat | | | | 3 | |
| TiO₂ | 1 | | 1 | | 1 |
| Ethylhexylmethoxycinnamat | 5 | 3 | 5 | | 3 |
| Ethylhexyltriazon | | 1 | | | |
| Ethylhexylcyanodiphenylacrylat (Octocrylen) | | | | 5 | 3 |
| Butylmethoxydibenzoylmethan | 1 | | | | |
| Bis-Ethylhexyloxyphenolmethoxyphenyltriazine | | 1 | | | |
| Ethylhexylsalicylat | 1 | | | | |
| 2-Hydroxy-4-Methoxy-benzophenon (Oxybenzone) | | | 2 | 3 | 2 |
| Phenylbenzimidazol sulfonsäure | | | | 2 | |
| Ubichinon (Q10) | | | 0,03 | | |
| Tocopherylacetat | | | 1 | | 0,3 |
| Citronensäure, Natriumsalz | | 0.1 | | | |
| Creatin | 0,5 | | | | |
| Natrium-Ascorbylphosphat | | | | | 0,1 |
| Phenoxyethanol | 0,3 | | 0,3 | 0,2 | 0,2 |
| p-Hydroxybenzoesäurealkylester (Paraben) | 0,6 | 0,3 | 0,2 | 0,3 | 0,3 |
| Hexamidindiisethionat | | 0,04 | | | |
| Diazolidinylharnstoff | 0,25 | | 0,1 | 0,2 | 0,1 |
| 1,3-Dimethylol-5,5-dimethyl-hydantoin(DMDM Hydantoin) | | 0,2 | | | |
| lodopropynylbutylcarbamat | | 0,1 | | | |
| Ethanol (vergällt) | | 2 | | | |
| Xanthan Gummi | | | 0,1 | | |
| Polyacrylsäure (Carbomer) | 0,05 | | | | 0,1 |
| Ammoniumpolyacryloyldimethyltaurate | 0,4 | | | 0,3 | |
| Ammoniumacryloyldimethyltaurate/ Vinylpyrrolidoncopolymere | | 0,5 | 0,3 | | |
| Aluminium Stärke Octenylsuccinate | | | | | 0,5 |
| Glycerin | 10 | 6 | 7 | 7 | 5 |
| Butylenglycol | | 1 | | 1 | |
| wasser- und/oder öllösliche Farbstoffe | 0,05 | | | | |
| Füllstoffe/ Additive (Distärkephosphat, SiO₂, BHT, Talkum, Aluminiumstearat) | 0,1 | 1 | 0,2 | 0,5 | 0,05 |
| Parfüm | q.s. | q.s. | q.s. | q.s. | q.s. |
| Wasser | ad 100 | ad 100 | ad 100 | ad 100 | ad 100 |

### Beispiel 6: Hydrodispersion/Gelcreme

| | |
|---|---|
| Cetylalkohol | 2 |
| Sheabutter | 1 |
| Caprylsäure/Caprinsäure Triglycerid | 2 |
| Octyldodecanol | 1 |
| Octamethyltetrasiloxan (Cyclomethicon) | 5 |
| Dimethylpolysiloxan (Dimethicon) | 1 |
| Polydecen | 2 |
| Ethylhexylmethoxycinnamat | 3 |
| Bis-Ethylhexyloxyphenol-methoxyphenyltriazin | 0,5 |
| Natriumascorbylphosphat | 0,05 |
| Iminodisuccinat | 0,2 |
| Ubichinon | 0,05 |
| Phenoxyethanol | 0,3 |
| p-Hydroxybenzoesäurealkyleste (Paraben) | 0,4 |
| Vernetztes Alkylacrylat (Alkylacrylate Crosspolymer) | 0,2 |
| Glycerin | 5 |
| Parfüm | q.s. |
| Wasser | ad 100 |

### B. Beispiele für die Zusammensetzung der Kapselhülle

### (Angaben in Massenprozent bezogen auf das Gesamtgewicht der Hülle)

### Beispiele 7-9: Wachshülle

| **Beispiel** | **7** | **8** | **9** |
|---|---|---|---|
| Myristylmyristat | | | 5 |
| Ceresin | | | 4 |
| Cera Alba | | | |
| Shea Butter | | | 7,5 |
| hydriertes Kokosfett | 10 | | 5 |
| Cera Microcristallina | | | 5 |
| Glycerylstearatcitrat | | | |
| Octyldodecanol | 15 | | 5 |
| Methylpalmitat | | 25 | |
| Ozokerit | | 10 | 5 |
| Hydriertes Polydecen | 15 | | |
| Polyethylene | 15 | 5 | |
| PEG-45/Dodecylglykol-Copolymer | 2 | | |
| Polyglyceryl-3-Diisostearat | 3 | | |
| Lanolin Alkohol | | 1 | 2 |
| Polyglyceryl-3-Diisostearat | | 1 | |
| PEG-40-Sorbitanperisostearat | | 3 | |
| Magnesiumstearat | | | 0,4 |
| Aluminiumstearat | | | 0,1 |
| Magnesiumsulfat | 1 | | |
| Natriumchlorid | | 1 | 1 |
| Konservierungsmittel | q. s. | q. s. | q. s. |
| Parfum | q. s. | q. s. | q. s. |
| Wasser | ad 100 | ad 100 | ad 100 |

### Beispiele 10-14: Wachshülle

| **Beispiel** | **10** | **11** | **12** | **13** | **14** |
|---|---|---|---|---|---|
| Myristylmyristat | | | 13 | | 5 |
| Ceresin | 5 | | | | 2 |
| Cera Alba | | | 10 | | |
| Shea Butter | | 10 | | 11 | |
| hydriertes Kokosfett | | 10 | | | |
| Cera Microcristallina | 5 | 3,5 | | 5 | |
| Octyldodecanol | | 10 | | 5 | 9 |
| Methylpalmitat | 25 | | 5 | 7 | 20 |
| Ozokerit | | 10 | | 5 | |
| Hydriertes Polydecen | | | | 2 | |
| Polyethylene | 5,5 | | | | 4 |
| Polyglyceryl-3-Diisostearat | | | | 3 | |
| Lanolin Alkohol | 0,6 | | 0,5 | 0,5 | |
| Magnesiumstearat | 0,4 | | | 0,5 | |
| Aluminiumstearat | 0,01 | | | | |
| Polyglyceryl-3 Diisostearat | 2,5 | | | | |
| PEG-30-Dipolyhydroxystearat | | 1,5 | | | |
| PEG-7 hydrierstes Ricinusöl | | | 0,5 | | |
| Polyglyceryl-2 Dipolyhydroxystearat | | | | 1 | |
| Bornitrid | | | | | 5 |
| Magnesiumsulfat | 1 | | 1 | | 1 |
| Natriumchlorid | | | | 1 | |
| Konservierungsmittel | q. s. | q. s. | q. s. | q. s. | q. s. |
| Parfum | q. s. | q. s. | q. s. | q. s. | q. s. |
| Wasser | ad 100 | ad 100 | ad 100 | ad 100 | ad 100 |

### Beispiele 15-17: Wachshülle

| **Beispiel** | **15** | **16** | **17** |
|---|---|---|---|
| Myristylmyristat | | | 5 |
| Cera Alba | | | |
| Shea Butter | | | 7,5 |
| hydriertes Kokosfett | 10 | | 9 |
| Cera Microcristallina | | | 5 |
| Glycerylstearatcitrat | | | |
| Methylpalmitat | | 10 | |
| Cetylrizinoleat | | 15 | 5 |
| Ozokerit | | 10 | 5 |
| Hydriertes Polydecen | 15 | | |
| Polyethylene | 15 | 5 | |
| Glycerylstearat | 2 | | |
| Polyglyceryl-3-Diisostearat | 3 | | |
| Lanolin Alkohol | | 1 | 2 |
| Polyglyceryl-3-Diisostearat | | 1 | |
| Sorbitanstearat | 1 | 3 | |
| Magnesiumstearat | | | 0,4 |
| Aluminiumstearat | | | 0,1 |
| Magnesiumsulfat | 1 | | |
| Natriumchlorid | | 1 | 1 |
| TiO2 | | 2 | |
| Ethylhexylmethoxycinnamat | 5 | | |
| PVP | | 1 | |
| PVP-Hexadecen-Copolymer | | | |
| Methylhydroxypropylcellulose | 2 | | |
| Ethylcellulose | | | 1 |
| Butylmethoxydibenzoylmethan | 2 | | |
| Dimethiconcopolyol | | | |
| Glycerin | 10 | 5 | 15 |
| Konservierungsmittel | q. s. | q. s. | q. s. |
| Parfum | q. s. | q. s. | q. s. |
| Wasser | ad 100 | ad 100 | ad 100 |

### Beispiele 18-21: Wachshülle

| **Beispiel** | **18** | **19** | **20** | **21** |
|---|---|---|---|---|
| Myristylmyristat | | | 13 | |
| Cera Alba | | | 10 | |
| Shea Butter | | 10 | | 11 |
| hydriertes Kokosfett | | 10 | | |
| Cera Microcristallina | 5 | 3,5 | | 5 |
| Octyldodecanol | | 10 | | 5 |
| Methylpalmitat | 30 | | 5 | 7 |
| Ozokerit | | 10 | | 5 |
| Hydriertes Polydecen | | | | 2 |
| Polyethylene | 5,5 | | | |
| Polyglyceryl-3-Diisostearat | 2,5 | 3 | | 3 |
| Lanolin Alkohol | 0,6 | | 0,5 | 0,5 |
| Magnesiumstearat | 0,4 | | | |
| Aluminiumstearat | 0,01 | | | |
| Bentonit | | | | 1 |
| Cetearylalkohol | | 1,5 | | |
| PEG-7 hydrierstes Ricinusöl | | | 0,5 | |
| Polyglyceryl-2 Dipolyhydroxystearat | | | | 1 |
| TiO2 | 1 | | | |
| Ethylhexylmethoxycinnamat | 3 | | 2 | |
| PVP | | | | |
| PVP-Hexadecen-Copolymer | 1 | | | |
| Methylhydroxypropylcellulose | | 1 | | |
| Ethylcellulose | 1 | 1 | 1 | |
| Butylmethoxydibenzoylmethan | 2 | | 1 | |
| Dimethiconcopolyol | | | | 1 |
| Glycerin | 5 | 3 | | 1 |
| Magnesiumsulfat | 1 | | 1 | |
| Natriumchlorid | | | | 1 |
| Konservierungsmittel | q. s. | q. s. | q. s. | q. s. |
| Parfum | q. s. | q. s. | q. s. | q. s. |
| Wasser | ad 100 | ad 100 | ad 100 | ad 100 |

Beispiele für besonders bevorzugte Rezepturbeispiele für W/O-Hüllmassen (Angaben in Gew.% bezogen auf die jeweilige Masse)

| **Beispiel** | **22** |
|---|---|
| Methylpalmitat | 15,0 |
| Mikrokristallines Wachs | 10,0 |
| Polyethylen | 5,0 |
| PEG-40 Sorbitan Perisostearate | 2,0 |
| Eucerit | 0,5 |
| Polyglyceryl-3 Diisostearate | 1,0 |
| Glycerin | 5,0 |
| MgSO₄ | 0,2 |
| Iodopropynyl Butylcarbamate | q.s. |
| Parabene | q.s. |
| Parfum | q.s. |
| Wasser | ad 100 |

| **Beispiel** | **23** |
|---|---|
| Methylstearat | 20,0 |
| Mikrokristallines Wachs | 8,0 |
| Carnaubawachs | 7,0 |
| C12-C15 Alkylbenzoat | 3,0 |
| Polyglyceryl-2 Dipolyhydroxystearate | 2,0 |
| Polyglyceryl-3 Diisostearate | 1,0 |
| Glycerin | 6,0 |
| MgSO₄ | 0,3 |
| hydrophobisierte Silica | q.s. |
| Phenoxyethanol | q.s. |
| Parabene | q.s. |
| Parfum | q.s. |
| Wasser | ad 100 |

| **Beispiel** | **24** |
|---|---|
| Ethylpalmitat | 18,0 |
| C18-38 Alkyl Hydroxystearoyl Stearate | 5,0 |
| Hydriertes Kokosfett | 10,0 |
| Macadamiaöl | 5,0 |
| Polyglyceryl-2 Dipolyhydroxystearate | 2,0 |
| Polyglyceryl-3 Diisostearate | 1,0 |
| Glycerin | 4,0 |
| MgSO₄ | 0,3 |
| Sorbinsäure | q.s. |
| Parabene | q.s. |
| Parfum | q.s. |
| Wasser | ad 100 |

| **Beispiel** | **25** |
|---|---|
| Ethylstearat | 15,0 |
| Ceresin | 10,0 |
| Cetylpalmitat | 5,0 |
| Paraffinöl | 5,0 |
| Polyglyceryl-2 Dipolyhydroxystearate | 2,0 |
| Polyglyceryl-3 Diisostearat, | 1,0 |
| Glycerin | 8,0 |
| MgSO₄ | 0,3 |
| Milchsäure | q.s. |
| Aluminum Starch Octenylsuccinate | q.s. |
| Parabene | q.s. |
| Parfum | q.s. |
| Wasser | ad 100 |

| **Beispiel** | **26** |
|---|---|
| Myristyllactat | 25,0 |
| Ceresin | 10,0 |
| Myristylmyristat | 5,0 |
| Polyethylen | 3,0 |
| Shea Butter | 1,0 |
| Butyl Methoxydibenzoylmethane | 1,0 |
| PEG-30 Dipolyhydroxystearate | 3,0 |
| Glycerin | 10,0 |
| MgSO₄ | 0,3 |
| Nylon | q.s. |
| lodopropynyl Butylcarbamate | q.s. |
| Parabene | q.s. |
| Parfum | q.s. |
| Na-EDTA | q.s. |
| Wasser | ad 100 |
| | |

| **Beispiel** | **27** |
|---|---|
| Cetyllactat | 18,0 |
| Ceresin | 2,0 |
| Mikrokristallines Wachs | 4,0 |
| Polyethylen | 6,0 |
| Ethylhexyl Methoxycinnamate | 1,0 |
| PEG-30 Dipolyhydroxystearate | 3,0 |
| Glycerin | 6,0 |
| MgO₄ | 0,3 |
| hydrophobisierte Silica | q.s. |
| DMDM Hydantoin | q.s. |
| Parabene | q.s. |
| Parfum | q.s. |
| Wasser | ad 100 |

| **Beispiel** | **28** |
|---|---|
| Lauryl PCA | 20,0 |
| Ceresin | 6,0 |
| Cetylpalmitat | 3,0 |
| Candelillawachs | 1,0 |
| Dimethicone | 2,0 |
| PEG-30 Dipolyhydroxystearate | 4,0 |
| Glycerin | 6,0 |
| MgSO₄ | 0,3 |
| hydrophobisierte Silica | q.s. |
| Phenoxyethanol | q.s. |
| Parabene | q.s. |
| Parfum | q.s. |
| Wasser | ad 100 |
| | |

| **Beispiel** | **29** |
|---|---|
| Myristylmyristat | 11,0 |
| hydriertes Koksfett | 9,0 |
| Mikrokristallines Wachs | 5,0 |
| Polyethylen | 3,0 |
| Dicaprylylcarbonat | 5,0 |
| Polyglyceryl-2 Dipolyhydroxystearate | 1,0 |
| Polyglyceryl-3 Diisostearate | 2,0 |
| Glycerin | 4,0 |
| MgSO₄ | 0,3 |
| Mica | q.s. |
| Phenoxyethanol | q.s. |
| Parabene | q.s. |
| Parfum | q.s. |
| Wasser | ad 100 |

Darüber hinaus können die Hülle oder Füllung unabhängig voneinander Hilfsstoffe wie UV-Filter, Wirkstoffe, Sensorikadditive, Verdicker, Gelbildner, Farbstoffe, Farb-, Effekt-, oder UV-Pigmente Konservierungsmittel Antioxidantien, Komplexbildner Geschmacksstoffe, Vergällungsmittel oder Parfum enthalten.

Wie beschrieben können die verschiedenen Füllungen von einer der beispielhaft aufgeführten Hülle umgeben werden. Die Auswahl der beispielhaft dargestellten Füllungen und Hüllen ist vom jeweiligen Anwendungszweck abhängig.

## Patentansprüche

1. Kosmetische und/oder dermatologische Kapsel für kosmetische und/oder dermatologische Inhaltsstoffe umfassend
a. eine Kapselhülle, die fest, halbfest und/oder formstabil ist, im Wesentlichen aus Wachsen, Emulgatoren, natürlichen und/oder synthetischen Polymeren und/oder Mischungen daraus besteht und Wasser enthält,
b. eine Füllung, umfassend ein oder mehrere feste, halbfeste, pastöse und/oder flüssige kosmetische und/oder dermatologische Inhaltsstoffe,
wobei die Kapsel überwiegend sphärische, runde oder elipsoide Formen mit einem Volumen von 0,1 bis 20 ml aufweist
**dadurch gekennzeichnet, dass**
die Polymere gewählt werden aus der Gruppe Celluloseether, Polyvinylpyrrolidon, Polyacrylate, Polymethacrylate, Polyethylene, Nylon und/oder Eudragit.

2. Kapsel nach Anspruch 1 **dadurch gekennzeichnet, dass** die Kapselhülle bis zu einer Temperatur von mindestens 35°C fest, halbfest und/oder formstabil ist.

3. Kapsel nach einem der Ansprüche 1 oder 2 **dadurch gekennzeichnet, dass** die Kapselhülle aus Wachsen, gewählt aus mikrokristallinen Wachsen, Paraffinwachsen, Esterwachsen, Glyceridwachsen, und/oder Fettalkoholen, festen Emulgatoren und deren Mischungen besteht.

4. Kapsel nach Anspruch 3 **dadurch gekennzeichnet, dass** die Wachse gewählt werden aus der Gruppe Cetylpalmitat, Cetylrizinoleat, Bienewachs, hydrierte Kokosglyceride, Methylpalmitat, Methylstearat, Myristyllactat, Cetyllactat, Stearyllactat, Candelillawachs, Carnaubawachs, Paraffinwachs, Ceresin, Ozokerit, Myristylmyristat, Tripalmitin, Tribehenin, Glycerylpalmitostearat, hydriertes Rapsöl und/oder C15-C40 Alkylstearylstearat, bevorzugt Ceresin und/ oder Ozokerit.

5. Kapsel nach Anspruch 1 bis 4 **dadurch gekennzeichnet, dass** die Hüllzusammensetzung Bestandteile und/oder Bestandteilkombinationen mit möglichst scharfen Schmelzpunkten oder engen Schmelzbereichen bei ca. 30°C enthält.

6. Kapsel nach einem der Ansprüche 1 bis 5. **dadurch gekennzeichnet, dass** die Kapselhülle zusätzlich Wasser zu einem Anteil von 50 bis 60 Gew.% und/oder Polyole enthält.

7. Kapsel nach einem der vorstehenden Ansprüche **dadurch gekennzeichnet, dass** die Kapselhülle bei Raumtemperatur flüssige Lipiden oder flüssige Mischungen davon enthält und durch Einbau von Wassertröpfchen verfestigt ist.

8. Kapsel nach einem der vorstehenden Ansprüche **dadurch gekennzeichnet, dass** die Kapselhülle aus einer formstabilen Lipid-/Emulgatormischung besteht, die dispergiertes Wasser mit einer Tröpfchengröße unter 50 Mikrometer enthält.

9. Kapsel nach einem der vorstehenden Ansprüche **dadurch gekennzeichnet, dass** die Kapselhülle oberflächenaktive Substanzen, bevorzugt W/O-Emulgatoren zugesetzt werden, bevorzugt gewählt aus der Gruppe verzweigte oder unverzweigte, gesättigte oder ungesättigte Fettsäuren mit 12 bis 26 Kohlenstoffatomen, Polyglyceryl-3 Dilsostearat, Polyglyceryl-4 Isostearat, Polyglyceryl-2 Dipolyhydroxystearat, Cetyl PEG/PPG-10-1- Dimethicone, PEG-30 Dipolyhydroxystearat, PEG-40 Sorbitanperisostearat, Cetyldimethiconecopolyol, PEG-7 Hydrogenated Castor Oil, PEG 45/Dodecylglycolcopolymer, PEG 22/Dodeceylglycolcopolymer, Pentaerythritylisostearat, Isostearyldiglycerylsuccinat, Sorbitanisostearat, Polyglyceryl-2 Sesquiisostearat, Glycerylisostearat, Sorbitanstearat, Glycerylstearat, PEG-25 Hydrogenated Castor Oil, PEG-40 Sorbitanperoleat, Sorbitanoleat, PEG-40 Sorbitanperisostearat, Polyglyceryl-3 Oleat, Polyglyceryl-2 Sesquioleat und Polyglyceryl-4 Isostearat und/oder Bienenwachse.

10. Kapsel nach einem der vorstehenden Ansprüche **dadurch gekennzeichnet, dass** die Kapsel einen durchschnittlichen Durchmesser bis 40 mm aufweisen.

11. Kapsel nach einem der vorstehenden Ansprüche **dadurch gekennzeichnet, dass** die Kapselhülle eine Dicke von 0,001 bis 3 mm, bevorzugt 0,01 bis 2 mm, besitzt.

12. Kapsel nach einem der vorstehenden Ansprüche **dadurch gekennzeichnet, dass** die Füllung zusätzlich Wasser enthalt.

13. Kapsel nach einem der vorstehenden Ansprüche **dadurch gekennzeichnet, dass** der oder die Inhaltsstoffe der Füllung gewählt werden aus der Gruppe wasserhaltiger Zubereitungen, O/W-, W/O-, W/O/W-Emulsionen, Gele, Hydrodispersionen, Tenside in fester oder flüssiger Form, Mikroemulsionen und/oder Mischungen daraus.

14. Kapsel nach Anspruch 13 **dadurch gekennzeichnet, dass** die Füllung eine O/W-Emulsion ist.

15. Kapsel nach Anspruch 13 oder 14 **dadurch gekennzeichnet, dass** die Hülle aus einer W/O-Emulsion und/oder Wachsen und die Füllung aus O/W-Emulsionen, wasserhaltigen Zusammensetzungen, Hydrogelen und/oder Hydrocolloiden besteht.

16. Kapsel nach Anspruch 13 **dadurch gekennzeichnet, dass** die Füllung waschaktive Substanzen und/oder Tenside in fester oder flüssiger Form enthält und die Hüllbdestandteile Wachse umfassen.

17. Kapsel nach einem der vorstehenden Ansprüche **dadurch gekennzeichnet, dass** die Kapselhülle und/oder die Füllung zusätzlich Hilfsstoffe, UV-Filter, Pigmente, Wirkstoffe, Farbstoffe, Sensorikadditive, Verdicker, Gelbildner, Konservierungsmittel, Antioxidantien, Komplexbildner, Geschmacksstoffe, Vergällungsmittel, Wirkstoffe und/oder Parfum enthalten.

18. Kapsel nach einem der Ansprüche 1 bis 17 **dadurch gekennzeichnet, dass** die Kapselhülle
- beim Verreiben und/oder Verteilen auf der Haut und/oder dem Haar schmilzt und/oder
- aufgrund von Scherkräften völlig oder teilweise flüssig wird und/oder
- sich in der Füllung und/oder den Hautsebumlipiden bzw. durch Vermischung von Füllung und Hüllmaterial auflöst und so nicht mehr, insbesondere als gesondert neben der Füllung vorliegender Bestandteil, wahrnehmbar ist.

19. Kosmetisches und/oder dermatologisches Produkt umfassend ein oder mehrere Kapseln nach einem der vorstehenden Ansprüche in einer Verpackung.

20. Kosmetisches und/oder dermatologisches Produkt nach Anspruch 19 **dadurch gekennzeichnet, dass** die Kapseln einzeln oder zu mehreren in einer Packung aus Papier, Metall oder Kunststoff verpackt sind.

21. Kosmetisches und/oder dermatologisches Produkt nach Anspruch 19 **dadurch gekennzeichnet, dass** die Kapseln einzeln oder zu mehreren in einer Blisterverpackung aus Papier, Metall oder Plastik verpackt sind.

22. Kosmetisches und/oder dermatologisches Produkt nach einem der Ansprüche 19 bis 21 **dadurch gekennzeichnet, dass** die Kapseln vorportioniert in einer Blisterverpackung zur Einzelentnahme verpackt sind.

23. Kosmetisches und/oder dermatologisches Produkt nach einem der Ansprüche 19 bis 21 **dadurch gekennzeichnet, dass** die Kapseln in einem Spendersystem verpackt sind.

24. Kosmetisches und/oder dermatologisches Produkt nach einem der Ansprüche 19 bis 23 bestehend aus mehreren Kapseln, von denen mindestens zwei sich in ihrem Aussehen, insbesondere ihrer Farbe unterscheiden, unterschiedliche Inhaltsstoffe aufweisen und/oder verschiedenen Zwecken dienen.

25. Kosmetisches und/oder dermatologisches Produkt nach einem der Ansprüche 19 bis 24 **dadurch gekennzeichnet, dass** die Kapseln alle oder teilweise einzeln mit Folie, Papier und/oder einem Beschichtungsmaterial umhüllt in einer gemeinsamen Verpackung vorliegen.

26. Verfahren zur Applikation kosmetischer Kapseln nach einem der Ansprüche 1 bis 18 auf der Haut und/oder dem Haar indem die Kapselhülle
- beim Verreiben und/oder Verteilen der Zubereitung auf der Haut und/oder dem Haar schmilzt und/oder
- aufgrund von Scherkräften völlig oder teilweise flüssig wird und/oder
- sich in der Füllung und/oder den Hautsebumlipiden bzw. durch Vermischung von Füllung und Hüllmaterial auflöst und so nicht mehr, insbesondere als gesondert neben der Füllung vorliegender Bestandteil, wahrnehmbar ist.

27. Verfahren zur Applikation kosmetischer Zubereitungen nach Anspruch 26 **dadurch gekennzeichnet, dass** eine oder mehrere Kapseln aus einer Verpackung entnommen und anschließen durch Reiben auf der Haut und/oder im Haar verteilt werden.

28. Verfahren zur Herstellung kosmetischer und/oder dermatologischer Kapseln nach einem der Ansprüche 11 bis 18 **dadurch gekennzeichnet, dass** die Füllung eingefroren wird, dann in aufgeschmolzenes, bei mindestens 35 °C fest werdendes Hüllmaterial getaucht wird, im Kugelcoater mit Hüllmaterial überzogen wird oder das Füllmaterial in verflüssigter Form mittels Wirbelschicht- oder Fließbettverfahren aufgesprüht wird und anschließend aushärtet, wodurch sich um die Füllung eine feste oder halbfeste geschlossene Hülle bildet und anschließend die sich gebildeten Kapseln nach Erreichen der gewünschten Hülldicke und Form aus der Schmelze entfernt werden.

29. Verfahren zur Herstellung kosmetischer und/oder dermatologischer Kapseln nach einem der Ansprüche 1 bis 18 **dadurch gekennzeichnet, dass** aus geschmolzenem Hüllmaterial Hohlkugeln hergestellt werden, die gegebenenfalls durch ein Loch In der Kapselwand mit Füllmaterial befüllt werden und anschließend das Loch durch einen Pfropfen aus Hüllmaterial verschlossen wird.

30. Verfahren zur Herstellung kosmetischer und/oder dermatologischer Kapseln nach einem der Ansprüche 1 bis 18 **dadurch gekennzeichnet, dass** zunächst halbe Hohlkugeln gegossen werden, welche gegebenenfalls einzeln befüllt, dann zur Deckung gebracht und abschließend durch thermische Behandlung miteinander verschmolzen werden.

31. Verfahren zur Herstellung kosmetischer und/oder dermatologischer Kapseln nach einem der Ansprüche 1 bis 18 **dadurch gekennzeichnet, dass** zwei Halbkugeln hergestellt werden, wobei eine oder beide ein Loch zu späteren Befüllung aufweisen, dann zu einer Hohlkugel verschweißt und abschlleßend gegebenenfalls durch das Füll-Loch mit der Füllung befüllt werden.

## Claims

1. Cosmetic and/or dermatological capsule for cosmetic and/or dermatological ingredients comprising:
a. a capsule shell, which is solid, semi-solid and/or dimensionally stable, essentially consists of waxes, emulsifiers, natural and/or synthetic polymers and/or mixtures thereof, and comprises water,
b. a filling, comprising one or more solid, semi-solid, pasty and/or liquid cosmetic and/or dermatological ingredients,
where the capsule has predominantly spherical, round or ellipsoidal shapes with a volume of from 0.1 to 20 ml,
**characterized in that**
the polymers are selected from the group cellulose ethers, polyvinylpyrrolidone, polyacrylates, polymethacrylates, polyethylenes, nylon and/or eudragit.

2. Capsule according to Claim 1, **characterized in that** the capsule shell is solid, semi-solid and/or dimensionally stable up to a temperature of at least 35°C.

3. Capsule according to one of Claims 1 and 2, **characterized in that** the capsule shell consists of waxes selected from microcrystalline waxes, paraffin waxes, ester waxes, glyceride waxes, and/or fatty alcohols, solid emulsifiers and mixtures thereof.

4. Capsule according to Claim 3, **characterized in that** the waxes are selected from the group cetyl palmitate, cetyl ricinoleate, beeswax, hydrogenated cocoglycerides, methyl palmitate, methyl stearate, myristyl lactate, cetyl lactate, stearyl lactate, candelilla wax, carnauba wax, paraffin wax, ceresin, ozokerite, myristyl myristate, tripalmitin, tribehenin, glyceryl palmitostearate, hydrogenated rapeseed oil and/or C15-C40 alkylstearyl stearate, preferably ceresin and/or ozokerite.

5. Capsule according to Claim 1 to 4, **characterized in that** the shell composition comprises constituents and/or constituent combinations with the sharpest possible melting points or narrow melting ranges at ca. 30°C.

6. Capsule according to one of Claims 1 to 5, **characterized in that** the capsule shell additionally comprises water to a fraction of 50 to 60% by weight and/or polyols.

7. Capsule according to one of the preceding claims, **characterized in that** the capsule shell comprises lipids which are liquid at room temperature, or liquid mixtures thereof, and is solidified by incorporating water droplets.

8. Capsule according to one of the preceding claims, **characterized in that** the capsule shell consists of a dimensionally stable lipid/emulsifier mixture which comprises dispersed water with a droplet size below 50 micrometres.

9. Capsule according to one of the preceding claims, **characterized in that** to the capsule shell are added surface-active substances, preferably W/O emulsifiers, preferably selected from the group branched or unbranched, saturated or unsaturated fatty acids having 12 to 26 carbon atoms, polyglyceryl-3 diisostearate, polyglyceryl-4 isostearate, polyglyceryl-2 dipolyhydroxystearate, cetyl PEG/PPG-10-1-dimethicones, PEG-30 dipolyhydroxystearate, PEG-40 sorbitan perisostearate, cetyldimethicone copolyol, PEG-7 hydrogenated castor oil, PEG 45/dodecyl glycol copolymer, PEG 22/dodecyl glycol copolymer, pentaerythrityl isostearate, isostearyl diglyceryl succinate, sorbitan isostearate, polyglyceryl-2 sesquiisostearate, glyceryl isostearate, sorbitan stearate, glyceryl stearate, PEG-25 hydrogenated castor oil, PEG-40 sorbitan peroleate, sorbitan oleate, PEG-40 sorbitan perisostearate, polyglyceryl-3 oleate, polyglyceryl-2 sesquioleate and polyglyceryl-4 isostearate and/or beeswaxes.

10. Capsule according to one of the preceding claims, **characterized in that** the capsule has an average diameter up to 40 mm.

11. Capsule according to one of the preceding claims, **characterized in that** the capsule shell has a thickness of from 0.001 to 3 mm, preferably 0.01 to 2 mm.

12. Capsule according to one of the preceding claims, **characterized in that** the filling additionally comprises water.

13. Capsule according to one of the preceding claims, **characterized in that** the ingredient or ingredients of the filling are selected from the group of water-containing preparations, O/W, W/O, W/O/W emulsions, gels, hydrodispersions, surfactants in solid or liquid form, microemulsions and/or mixtures thereof.

14. Capsule according to Claim 13, **characterized in that** the filling is an O/W emulsion.

15. Capsule according to Claim 13 or 14, **characterized in that** the shell consists of a W/O emulsion and/or waxes and the filling consists of O/W emulsions, water-containing compositions, hydrogels and/or hydrocolloids.

16. Capsule according to Claim 13, **characterized in that** the filling comprises washing-active substances and/or surfactants in solid or liquid form and the shell constituents comprise waxes.

17. Capsule according to one of the preceding claims, **characterized in that** the capsule shell and/or the filling additionally comprise auxiliaries, UV filters, pigments, active ingredients, dyes, sensory additives, thickeners, gel formers, preservatives, antioxidants, complexing agents, flavourings, denaturing agents, active ingredients and/or perfume.

18. Capsule according to one of Claims 1 to 17, **characterized in that** the capsule shell
- melts upon rubbing and/or spreading on the skin and/or the hair and/or
- becomes completely or partly liquid on account of shear forces and/or
- dissolves in the filling and/or the skin sebum lipids or as a result of mixing filling and shell material and is thus no longer perceptible, especially as a separate constituent present alongside the filling.

19. Cosmetic and/or dermatological product comprising one or more capsules according to one of the preceding claims in a packaging.

20. Cosmetic and/or dermatological product according to Claim 19, **characterized in that** the capsules are packaged individually or in multiples in a packaging made of paper, metal or plastic.

21. Cosmetic and/or dermatological product according to Claim 19, **characterized in that** the capsules are packaged individually or in multiples in a blister pack made of paper, metal or plastic.

22. Cosmetic and/or dermatological product according to one of Claims 19 to 21, **characterized in that** the capsules are packaged pre-portioned in a blister pack for individual removal.

23. Cosmetic and/or dermatological product according to one of Claims 19 to 21, **characterized in that** the capsules are packaged in a dispenser system.

24. Cosmetic and/or dermatological product according to one of Claims 19 to 23 consisting of a plurality of capsules, of which at least two differ in terms of their appearance, in particular their colour, have different ingredients and/or serve different purposes.

25. Cosmetic and/or dermatological product according to one of Claims 19 to 24, **characterized in that** the capsules are present in a common packaging, all or partly individually covered with film, paper and/or a coating material.

26. Method for the application of cosmetic capsules according to one of Claims 1 to 18 on the skin and/or the hair, in which the capsule shell
- melts upon rubbing and/or spreading the preparation on the skin and/or the hair and/or
- becomes completely or partly liquid on account of shear forces and/or
- dissolves in the filling and/or the skin sebum lipids or as a result of mixing filling and shell material and is thus no longer perceptible, especially as a separate constituent present alongside the filling.

27. Method for the application of cosmetic preparations according to Claim 26, **characterized in that** one or more capsules are removed from a packaging and are then spread on the skin and/or in the hair by rubbing.

28. Method for producing cosmetic and/or dermatological capsules according to one of Claims 11 to 18, **characterized in that** the filling is frozen, is then dipped into molten shell material which solidifies at at least 35°C, is coated with shell material in the Kugelcoater, or the filling material is sprayed on in liquefied form by means of a fluidized-bed or moving-bed method and is then hardened, as a result of which a solid or semi-solid closed shell forms around the filling and then the capsules formed are removed from the melt after reaching the desired shell thickness and shape.

29. Method for producing cosmetic and/or dermatological capsules according to one of Claims 1 to 18, **characterized in that** hollow spheres are produced from molten shell material which are optionally filled with filling material through a hole in the capsule wall and then the hole is closed by means of a grafting from shell material.

30. Method for producing cosmetic and/or dermatological capsules according to one of Claims 1 to 18, **characterized in that** firstly half hollow spheres are cast which are optionally filled individually, then brought to the covering and finally melted with one another by means of thermal treatment.

31. Method for producing cosmetic and/or dermatological capsules according to one of Claims 1 to 18, **characterized in that** two half-spheres are produced, where one or both have a hole for subsequent filling, then are welded to give one hollow sphere and, finally, are optionally filled with the filling through the filling hole.

## Revendications

1. Capsule cosmétique et/ou dermatologique pour composants cosmétiques et/ou dermatologiques, comprenant
a. une enveloppe de capsule, qui est solide, semi-solide et/ou de forme stable, est essentiellement constituée de cires, d'émulsifiants, de polymères naturels et/ou synthétiques et/ou de mélanges de ceux-ci et contient de l'eau,
b. une masse de remplissage, comprenant un ou plusieurs composants cosmétiques et/ou dermatologiques solides, semi-solides, pâteux et/ou liquides,
la capsule présentant essentiellement des formes sphériques, rondes ou ellipsoïdes, ayant un volume de 0,1 à 20 ml,
**caractérisée en ce que**
les polymères sont choisis dans le groupe constitué par des éthers de cellulose, la polyvinylpyrrolidone, des polyacrylates, des polyméthacrylates, des polyéthylènes, le nylon et/ou l'Eudragit.

2. Capsule selon la revendication 1, **caractérisée en ce que** l'enveloppe de la capsule est solide, semi-solide ou de forme stable jusqu'à une température d'au moins 35 °C.

3. Capsule selon la revendication 1 ou 2, **caractérisée en ce que** l'enveloppe de la capsule est constituée de cires, choisies parmi des cires microcristallines, des cires de paraffine, des cires d'esters, des cires de glycérides et/ou des alcools gras, des émulsifiants solides et des mélanges de ceux-ci.

4. Capsule selon la revendication 3, **caractérisée en ce que** les cires sont choisies dans le groupe constitué par le palmitate de cétyle, le ricinoléate de cétyle, la cire d'abeille, des glycérides de coco hydrogénés, le palmitate de méthyle, le stéarate de méthyle, le lactate de myristyle, le lactate de cétyle, la lactate de stéaryle, la cire de candelilla, la cire de carnauba, la cire de paraffine, la cérésine, l'ozokérite, le myristate de myristyle, la tripalmitine, la tribéhénine, le palmitostéarate de glycéryle, l'huile de colza hydrogénée et/ou un stéarate d'alkyl(C₁₅-C₄₀)stéaryle, de préférence la cérésine et/ou l'ozokérite.

5. Capsule selon l'une quelconque des revendications 1 à 4, **caractérisée en ce que** la composition d'enveloppe contient des constituants et/ou des associations de constituants à points de fusion les plus nets possibles ou domaines de fusion les plus étroits possibles à environ 30 °C.

6. Capsule selon l'une quelconque des revendications 1 à 5, **caractérisée en ce que** l'enveloppe de capsule en plus contient de l'eau en une proportion de 50 à 60 % en poids et/ou des polyols.

7. Capsule selon l'une quelconque des revendications précédentes, **caractérisée en ce que** l'enveloppe de capsule contient des lipides liquides à la température ambiante ou des mélanges liquides de ceux-ci et est consolidée par incorporation de gouttelettes d'eau.

8. Capsule selon l'une quelconque des revendications précédentes, **caractérisée en ce que** l'enveloppe de capsule est constituée d'un mélange de lipide/émulsifiant de forme stable, qui contient de l'eau dispersée ayant une taille de gouttelette inférieure à 50 µm.

9. Capsule selon l'une quelconque des revendications précédentes, **caractérisée en ce qu'**on ajoute à l'enveloppe de capsule des substances tensioactives, de préférence des émulsifiants E/H, de préférence choisis dans le groupe constitué par des acides gras saturés ou insaturés, ramifiés ou non ramifiés, ayant de 12 à 26 atomes de carbone, le polyglycéryl-3 diisostéarate, le polyglycéryl-4 isostéarate, le polyglycéryl-2 dipolyhydroxystéarate, la cétyl PEG/PPG-10-1- diméthicone, le PEG-30 dipolyhydroxystéarate, le PEG-40 sorbitan perisostéarate, le cétyldiméthiconecopolyol, l'huile de ricin hydrogénée PEG-7, le PEG 45/dodécylglycol copolymère, le PEG 22/dodécylglycol copolymère, le pentaerythrityl isostéarate, l'isostéaryldiglycéryl succinate, le sorbitan isostéarate, le polyglycéryl-2 sesquiisostéarate, le glycéryl isostéarate, le sorbitan stéarate, le glycéryl stéarate, l'huile de ricin hydrogénée PEG-25, le PEG-40 sorbitan peroléate, le sorbitan Oleate, le PEG-40 Sorbitan Perisostearate, le polyglycéryl-3 oléate, le polyglycéryl-2 sesquioléate et le polyglycéryl-4 isostéarate et/ou la cire d'abeille.

10. Capsule selon l'une quelconque des revendications précédentes, **caractérisée en ce que** la capsule présente un diamètre moyen de jusqu'à 40 mm.

11. Capsule selon l'une quelconque des revendications précédentes, **caractérisée en ce que** l'enveloppe de capsule présente une épaisseur de 0,001 à 3 mm, de préférence de 0,01 à 2 mm.

12. Capsule selon l'une quelconque des revendications précédentes, **caractérisée en ce que** la masse de remplissage contient en outre de l'eau.

13. Capsule selon l'une quelconque des revendications précédentes, **caractérisée en ce que** le ou les composants de la masse de remplissage est/sont choisi(s) dans le groupe constitué par des préparations contenant de l'eau, des émulsions H/E, E/H, E/H/E, des gels, des hydrodispersions, des tensioactifs, sous forme solide ou liquide, des microémulsions et/ou des mélanges de ceux-ci.

14. Capsule selon la revendication 13, **caractérisée en ce que** la masse de remplissage est une émulsion H/E.

15. Capsule selon la revendication 13 ou 14, **caractérisée en ce que** l'enveloppe consiste en une émulsion E/H et/ou des cires et la masse de remplissage consiste en des émulsions H/E, des compositions contenant de l'eau, des hydrogels et/ou des hydrocolloïdes.

16. Capsule selon la revendication 13, **caractérisée en ce que** la masse de remplissage contient des substances lavantes et/ou des tensioactifs sous forme solide ou liquide et les constituants de l'enveloppe comprennent des cires.

17. Capsule selon l'une quelconque des revendications précédentes, **caractérisée en ce que** l'enveloppe de capsule et/ou la masse de remplissage contient/contiennent en outre des charges, des filtres UV, des pigments, des substances actives, des colorants, des additifs sensoriels, des épaississants, des gélifiants, des conservateurs, des antioxydants, des complexants, des agents de sapidité, des dénaturants et/ou un parfum.

18. Capsule selon l'une quelconque des revendications 1 à 17, **caractérisée en ce que** l'enveloppe de capsule
- fond lors du passage avec frottement et/ou de la répartition sur la peau et/ou les cheveux et/ou
- se liquéfie partiellement ou totalement sous l'effet de forces de cisaillement et/ou
- se dissout dans la masse de remplissage et/ou les lipides du sébum de la peau et/ou par mélange de la masse de remplissage et du matériau d'enveloppe et ainsi aucun constituant, en particulier présent sous forme séparée en plus de la masse de remplissage, n'est plus décelable.

19. Produit cosmétique et/ou dermatologique, comprenant une ou plusieurs capsules selon l'une quelconque des revendications précédentes dans un emballage.

20. Produit cosmétique et/ou dermatologique selon la revendication 19, **caractérisé en ce que** les capsules sont conditionnées individuellement ou à plusieurs dans un emballage en papier, métal ou matière plastique.

21. Produit cosmétique et/ou dermatologique selon la revendication 19, **caractérisé en ce que** les capsules sont conditionnées individuellement ou à plusieurs dans un emballage thermoformé en papier, métal ou matière plastique.

22. Produit cosmétique et/ou dermatologique selon l'une quelconque des revendications 19 à 21, **caractérisé en ce que** les capsules sont pré-réparties dans un emballage thermoformé conçu pour le prélèvement individuel.

23. Produit cosmétique et/ou dermatologique selon l'une quelconque des revendications 19 à 21, **caractérisé en ce que** les capsules sont conditionnées dans un système distributeur.

24. Produit cosmétique et/ou dermatologique selon l'une quelconque des revendications 19 à 23, consistant en plusieurs capsules, dont au moins deux se distinguent par leur aspect, en particulier leur couleur, présentent des contenus différents et/ou servent à des fins différentes.

25. Produit cosmétique et/ou dermatologique selon l'une quelconque des revendications 19 à 24, **caractérisé en ce que** les capsules sont présentes dans un emballage commun, toutes ou en partie individuellement enveloppées avec un film, du papier et/ou un matériau d'enrobage.

26. Procédé pour l'application de capsules cosmétiques selon l'une quelconque des revendications 1 à 18 sur la peau et/ou les cheveux, consistant en ce que l'enveloppe de capsule
- fond lors du passage avec frottement et/ou de la répartition sur la peau et/ou les cheveux et/ou
- se liquéfie partiellement ou totalement sous l'effet de forces de cisaillement et/ou
- se dissout dans la masse de remplissage et/ou les lipides du sébum de la peau ou par mélange de la masse de remplissage et du matériau d'enveloppe et ainsi aucun constituant, en particulier présent sous forme séparée en plus de la masse de remplissage, n'est plus décelable.

27. Procédé pour l'application de préparations cosmétiques selon la revendication 26, **caractérisé en ce qu'**on prélève une ou plusieurs capsules d'un emballage et ensuite on la/les répartit par frottement sur la peau et/ou dans les cheveux.

28. Procédé pour la fabrication de capsules cosmétiques et/ou dermatologiques selon l'une quelconque des revendications 11 à 18, **caractérisé en ce qu'**on fait congeler la masse de remplissage, puis on la plonge dans du matériau d'enveloppe fondu, se solidifiant à au moins 35 °C, on l'enrobe avec du matériau d'enveloppe dans l'appareil d'enrobage à billes ou on pulvérise au moyen d'un procédé en lit fluidisé ou en lit tourbillonnaire la matière de remplissage sous forme liquéfiée, et ensuite on sèche, de sorte qu'une enveloppe solide ou semi-solide continue se forme autour de la masse de remplissage et ensuite, une fois que la forme et l'épaisseur désirées de l'enveloppe sont obtenues, on retire de la masse fondue les capsules formées.

29. Procédé pour la fabrication de capsules cosmétiques et/ou dermatologiques selon l'une quelconque des revendications 1 à 18, **caractérisé en ce qu'**à partir de matériau d'enveloppe fondu on produit des billes creuses, que l'on remplit de matière de remplissage à travers un trou dans la paroi de la capsule et ensuite on obture le trou par un bouchage en matériau d'enveloppe.

30. Procédé pour la fabrication de capsules cosmétiques et/ou dermatologiques selon l'une quelconque des revendications 1 à 18, **caractérisé en ce que** d'abord on coule des demi-sphères creuses qu'éventuellement on remplit individuellement, puis on les fait concorder et enfin on les fait fondre l'une avec l'autre par traitement thermique.

31. Procédé pour la fabrication de capsules cosmétiques et/ou dermatologiques selon l'une quelconque des revendications 1 à 18, **caractérisé en ce qu'**on fabrique deux demi-sphères, une ou les deux présentant un trou pour le remplissage ultérieur, puis on les soude en une sphère creuse et enfin éventuellement on les remplit avec la masse de remplissage par le trou de remplissage.
